# EUROPEAN PATENT APPLICATION

(11) **EP 3 379 593 A1**
(43) Date of publication of application: **26.09.2018**
(21) Application number: 16866358.1
(22) Date of filing: 16.11.2016
(51) Int. Cl.: H01L 51/50, C09K 11/06, C07C 211/58, C07D 307/91

(54) **ORGANIC ELECTROLUMINESCENCE ELEMENT**

(30) Priority: 17.11.2015 JP 2015224450
(71) Applicant: Hodogaya Chemical Co., Ltd., Chuo-ku Tokyo 104-0028 (JP)
(72) Inventor: SURUGA, Kazuyuki, Tokyo 104-0028 (JP); KASE, Kouki, Tokyo 104-0028 (JP); IZUMIDA, Junichi, Tokyo 104-0028 (JP); MOCHIDUKI, Syunji, Tokyo 104-0028 (JP)
(74) Representative: Müller-Boré & Partner Patentanwälte PartG mbB
(86) International application number: PCT/JP2016/083995
(87) International publication number: WO 2017/086357

(57) **Abstract**

The present invention provides an organic EL device having at least an anode, a hole-transporting layer, a luminous layer, an electron-transporting layer and a cathode arranged in this order, wherein the hole-transporting layer contains an arylamine compound represented by the following general formula (1). The organic EL device of the present invention has a high efficiency, drives on a low voltage, and features a specifically long life.

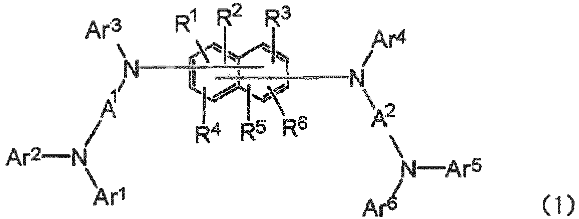

## Description

### Technical Field:

This invention relates to organic electroluminescent devices (hereinafter often called organic EL devices) which are spontaneously luminous devices that can be favorably used for various kinds of display devices. More specifically, the invention relates to organic EL devices using specific arylamine compounds (and specific anthracene derivatives).

### Background Art:

Organic EL devices are spontaneously luminous devices which feature higher brightness and higher legibility than those of liquid crystal devices enabling vivid display to be realized, and have, therefore, been vigorously studied.

In 1987, C. W. Tang et al. of Eastman Kodak have developed a device of a layer-laminated structure comprising various kinds of materials to bear individual roles, and have put an organic EL device using organic materials into a practical use. The above organic EL device is constituted by laminating layers of a fluorescent body capable of transporting electrons and of an organic material capable of transporting holes. Upon injecting both electric charges into the layer of the fluorescent body to emit light, a brightness of as high as 1000 cd/m² or more has now been attained with a voltage of not higher than 10 V (see a patent document 1 and a patent document 2).

So far, very many improvements have been made to put the organic EL device into practical use. For example, there has been put into practical use an electroluminescent device comprising an anode, a hole injection layer, a hole-transporting layer, a luminous layer, an electron-transporting layer, an electron injection layer and a cathode which are arranged in this order on a substrate more finely dividing the roles of the layers in the laminated-layer structure. The device is now achieving a high efficiency and a high durability.

To further improve the luminous efficiency, attempts have been made to utilize triplet excitons and study has been forwarded to utilize a phosphorescent luminous compound. Devices have, further, been developed utilizing the emission of light based on the thermally activated delayed fluorescence (TADF). In 2011, Adachi et al. of Kyushu University have realized an external quantum efficiency of 5.3% by using a device comprising the thermally activated delayed fluorescent material.

The luminous layer is, usually, prepared by doping an electric charge-transporting compound called host material with a fluorescent luminous compound, a phosphorescent luminous compound or a material that emits delayed fluorescence. Selection of the organic materials in the organic EL device seriously affects the properties of the device, such as efficiency and durability.

In the organic EL device, the electric charges injected from the two electrodes recombine together in the luminous layer to emit light. In the organic EL device, therefore, what is important is how efficiently to hand both electric charges, i.e., holes and electrons, over to the luminous layer; i.e., the device must have an excellent carrier balance. Upon improving the probability of recombination of the holes and the electrons by improving the hole injection capability and by improving the electron-blocking property to block electrons that are injected through the cathode, and, further, confining the excitons formed in the luminous layer, it is allowed to attain a high luminous efficiency. Namely, the hole-transporting material plays an important role. Therefore, it has been desired to provide an hole-transporting material that has a high hole injection capability, a high hole mobility, a high electron-blocking property and a large durability against the electrons.

As for the life of the device, further, the heat resistance and amorphousness of the material also serve as important factors. The material having small heat resistance is subject to be thermally decomposed even at a low temperature due to the heat generated when the device is driven, and is deteriorated. The material having low amorphousness permits the thin film thereof to be crystallized in short periods of time and, therefore, the device to be deteriorated. Accordingly, the material to be used must have large heat resistance and good amorphousness.

N,N'-diphenyl-N,N'-di(α-naphthyl)benzidine (NPD) and various aromatic amine derivatives have heretofore been known as the hole-transporting materials for the organic EL devices (see a patent document 1 and a patent document 2). The NPD has a favorable hole-transporting capability but its glass transition point (Tg) that serves as an index of heat resistance is as low as 96°C. Under high temperature conditions, therefore, the NPD undergoes the crystallization and causes a decrease in the device characteristics. Further, some of the aromatic amine derivatives disclosed in the patent documents 1 and 2 have excellent hole mobilities of not less than 10⁻³ cm²/Vs but also have insufficient electron-blocking property. With the organic EL devices formed by using such aromatic amine derivatives, therefore, the electrons partly pass through the luminous layer and improvements in the luminous efficiency cannot be expected. To further improve the efficiency, therefore, it has been urged to obtain a material that has higher electron-blocking property, remains more stable in the form of a thin film and has higher heat resistance.

As a compound having improved properties such as heat resistance, hole injection property, hole-transporting property and electron-blocking property, there has been proposed an aromatic tertiary amine compound (compound A) represented by the following formula (see a patent document 3) .

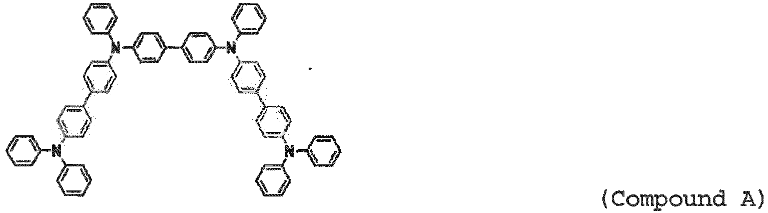

With the device in which the compound A is used for forming the hole injection layer, the hole-transporting layer or the electron-blocking layer, however, the heat resistance and luminous efficiency can be improved, which, however, are not still satisfactory. Besides, the device cannot be still driven on a sufficiently low voltage, the current efficiency is not satisfactory, either, and a problem still remains in regard to amorphousness. Therefore, it has been urged to provide a material that can attain higher amorphousness, that can be driven on a lower voltage and that can realize a higher luminous efficiency.

### Prior Art Documents:

### Patent Documents:

Patent document 1: JP-A-8-48656
Patent document 2: Japanese Patent No. 3194657
Patent document 3: International Laid-Open WO2012/117973
Patent document 4: International Laid-Open WO2011/059000
Patent document 5: International Laid-Open WO2003/060956
Patent document 6: Korean Patent Laid-Open No. 2013-0060157

### Outline of the Invention:

### Problems that the Invention is to Solve:

It is, therefore, an object of the present invention to provide an organic EL device which (1) has a high luminous efficiency and a high power efficiency, (2) drives on a low voltage, and (3) has a long life (high durability) by using in combination various materials for organic EL devices that feature excellent hole-injecting·transporting capability, electron-injecting·transporting capability, electron-blocking capability and stability or durability in the form of a thin film, the materials being so combined together as to effectively exhibit their specific properties.

### Means for Solving the Problems:

To achieve the above object, the present inventors have paid attention to that the arylamine type material has excellent hole-injecting and transporting capability, and excellent stability or durability in the form of a thin film, and have considered that a hole-transporting layer formed by using the arylamine compound would inject and transport the holes efficiently. The inventors have, further, paid attention to that a compound having an anthracene ring structure has excellent electron-injecting and transporting capability, and excellent stability or durability in the form of a thin film, and have considered that if an anthracene derivative having a specific structure is selected as a material of the electron-transporting layer, then the electrons could be efficiently injected and transported. The inventors, therefore, have fabricated a variety of organic EL devices by combining these hole-transporting material and electron-transporting material together in such a manner that a carrier balance could be attained, and have keenly evaluated the properties of the devices. As a result, the present invention was completed.

That is, according to the present invention, there is provided:
1) An organic EL device having at least an anode, a hole-transporting layer, a luminous layer, an electron-transporting layer and a cathode arranged in this order,
   wherein the hole-transporting layer contains an arylamine compound represented by the following general formula (1), 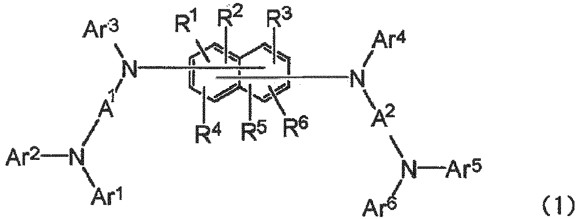 wherein,
   Ar¹ to Ar⁶ may be the same or different, and are aromatic hydrocarbon groups, aromatic heterocyclic groups or condensed polycyclic aromatic groups,
   A¹ and A² may be the same or different, and are divalent aromatic hydrocarbon groups, divalent aromatic heterocyclic groups or divalent condensed polycyclic aromatic groups, and
   R¹ to R⁶ may be the same or different, and are hydrogen atoms, deuterium atoms, fluorine atoms, chlorine atoms, cyano groups, nitro groups, alkyl groups having 1 to 6 carbon atoms, cycloalkyl groups having 5 to 10 carbon atoms, alkenyl groups having 2 to 6 carbon atoms, alkyloxy groups having 1 to 6 carbon atoms, cycloalkyloxy groups having 5 to 10 carbon atoms, aromatic hydrocarbon groups, aromatic heterocyclic groups, condensed polycyclic aromatic groups or aryloxy groups.

   Described below are preferred embodiments of the organic EL device of the present invention.
2) The arylamine compound is represented by the following general formula (1a), 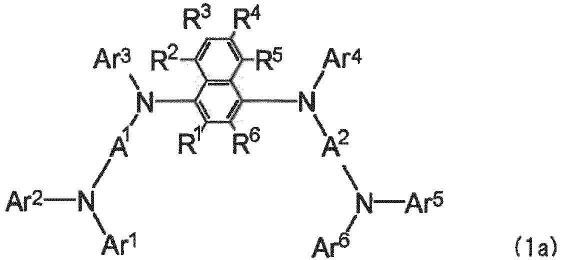 wherein,
   Ar¹ to Ar⁶, A¹, A² and R¹ to R⁶ are as defined in the above general formula (1).
3) The arylamine compound is represented by the following general formula (1b), 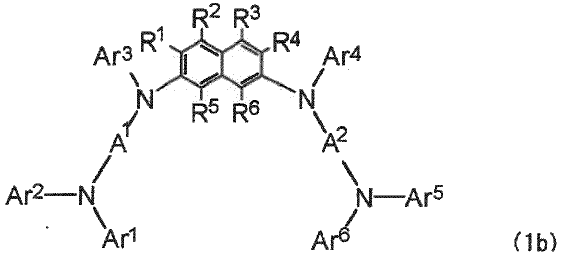 wherein,
   Ar¹ to Ar⁶, A¹, A² and R¹ to R⁶ are as defined in the above general formula (1).
4) The arylamine compound is represented by the following general formula (1c), 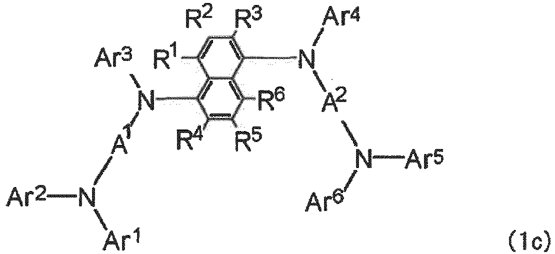 wherein,
   Ar¹ to Ar⁶, A¹, A² and R¹ to R⁶ are as defined in the above general formula (1).
5) The electron-transporting layer contains an anthracene derivative represented by the following general formula (2), 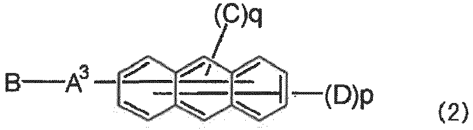 wherein,
   A³ is a divalent aromatic hydrocarbon group, a divalent aromatic heterocyclic group, a divalent condensed polycyclic aromatic group or a single bond,
   B is an aromatic heterocyclic group,
   C is an aromatic hydrocarbon group, an aromatic heterocyclic group or a condensed polycyclic aromatic group, and when there are two Cs, the two Cs may be the same or different,
   D may be the same or different, and is a hydrogen atom, a deuterium atom, a fluorine atom, a chlorine atom, a cyano group, a trifluoromethyl group, an alkyl group having 1 to 6 carbon atoms, an aromatic hydrocarbon group, an aromatic heterocyclic group or a condensed polycyclic aromatic group, and
   p is 7 or 8, and q is 1 or 2 under the condition that the sum of p and q is 9.
6) The anthracene derivative is represented by the following general formula (2a), 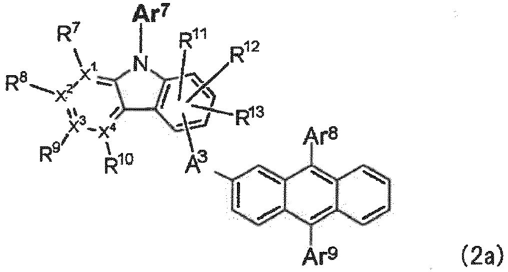 wherein,
   A³ is a divalent aromatic hydrocarbon group, a divalent aromatic heterocyclic group, a divalent condensed polycyclic aromatic group or a single bond,
   Ar⁷ to Ar⁹ may be the same or different, and are aromatic hydrocarbon groups, aromatic heterocyclic groups or condensed polycyclic aromatic groups,
   R⁷ to R¹³ may be the same or different, and are hydrogen atoms, deuterium atoms, fluorine atoms, chlorine atoms, cyano groups, nitro groups, alkyl groups having 1 to 6 carbon atoms, cycloalkyl groups having 5 to 10 carbon atoms, alkenyl groups having 2 to 6 carbon atoms, alkyloxy groups having 1 to 6 carbon atoms, cycloalkyloxy groups having 5 to 10 carbon atoms, aromatic hydrocarbon groups, aromatic heterocyclic groups, condensed polycyclic aromatic groups or aryloxy groups, and may be bonded to each other via a single bond, a methylene group, an oxygen atom or a sulfur atom to form a ring, and
   X¹ to X⁴ are, respectively, carbon atoms or nitrogen atoms, only any one of X¹ to X⁴ being the nitrogen atom and, in this case, the nitrogen atom does not have any R⁷ to R¹⁰ of hydrogen atom or a substituent.
7) The anthracene derivative is represented by the following general formula (2b), 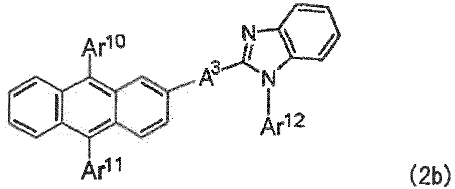 wherein,
   A³ is a divalent aromatic hydrocarbon group, a divalent aromatic heterocyclic group, a divalent condensed polycyclic aromatic group or a single bond, and
   Ar¹⁰ to Ar¹² may be the same or different, and are aromatic hydrocarbon groups, aromatic heterocyclic groups or condensed polycyclic aromatic groups.
8) The anthracene derivative is represented by the following general formula (2c), 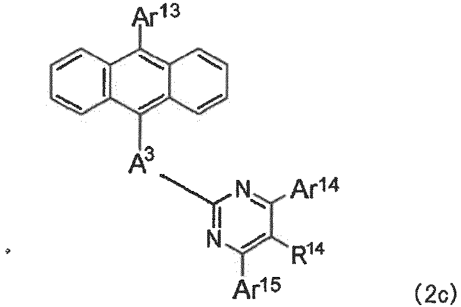 wherein,
   A³ is a divalent aromatic hydrocarbon group, a divalent aromatic heterocyclic group, a divalent condensed polycyclic aromatic group or a single bond,
   Ar¹³ to Ar¹⁵ may be the same or different, and are aromatic hydrocarbon groups, aromatic heterocyclic groups or condensed polycyclic aromatic groups, and
   R¹⁴ is a hydrogen atom, a deuterium atom, a fluorine atom, a chlorine atom, a cyano group, a nitro group, an alkyl group having 1 to 6 carbon atoms, a cycloalkyl group having 5 to 10 carbon atoms, an alkenyl group having 2 to 6 carbon atoms, an alkyloxy group having 1 to 6 carbon atoms, a cycloalkyloxy group having 5 to 10 carbon atoms, an aromatic hydrocarbon group, an aromatic heterocyclic group, a condensed polycyclic aromatic group or an aryloxy group.
9) The luminous layer contains a blue light-emitting dopant.
10) The blue light-emitting dopant is a pyrene derivative.
11) The luminous layer contains an anthracene derivative.
12) The luminous layer contains the anthracene derivative as a host material.
13) The anthracene derivative is represented by the following general formula (3), 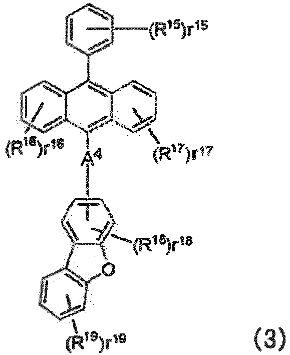 wherein,
   R¹⁵ to R¹⁹ may be the same or different, and are deuterium atoms, alkyl groups having 1 to 30 carbon atoms, alkenyl groups having 2 to 30 carbon atoms, alkinyl groups having 2 to 30 carbon atoms, cycloalkyl groups having 3 to 30 carbon atoms, cycloalkenyl groups having 5 to 30 carbon atoms, alkyloxy groups having 1 to 30 carbon atoms, aryloxy groups having 6 to 30 carbon atoms, alkylthio groups having 1 to 30 carbon atoms, arylthio groups having 5 to 30 carbon atoms, alkylamino groups having 1 to 30 carbon atoms, arylamino groups having 5 to 30 carbon atoms, aryl groups having 6 to 50 carbon atoms, aromatic heterocyclic groups having 2 to 50 carbon atoms, cyano groups, nitro groups, halogen atoms, amino groups, hydroxy groups or -CO-R²⁰ groups,
   R²⁰ is a hydrogen atom, a hydroxy group, an alkyloxy group having 1 to 6 carbon atoms or an aryloxy group having 6 to 30 carbon atoms,
   A⁴ is a divalent aromatic hydrocarbon group, a divalent condensed polycyclic aromatic group or a single bond,
   r¹⁵ is an integer of 0 to 5, r¹⁶, r¹⁷ and r¹⁹ are, respectively, integers of 0 to 4, and r¹⁸ is an integer of 0 to 3, and
   when R¹⁵ to R¹⁹ are bonded to the same benzene ring plurally, the plurality of the groups that are bonded may be the same or different.

According to the present invention, further, there are provided:
14) An organic EL device having an anode, a hole-transporting layer, an electron-blocking layer, a luminous layer, an electron-transporting layer and a cathode arranged in this order, wherein the electron-blocking layer contains an arylamine compound represented by the above-mentioned general formula (1);
15) An organic EL device having an anode, a hole injection layer, a hole-transporting layer, a luminous layer, an electron-transporting layer and a cathode arranged in this order, wherein the hole injection layer contains an arylamine compound represented by the above-mentioned general formula (1); and
16) An organic EL device having an anode, a hole-transporting layer, a luminous layer, an electron-transporting layer and a cathode arranged in this order, wherein the luminous layer contains an arylamine compound represented by the above-mentioned general formula (1).

### Effects of the Invention:

The arylamine compound having four triarylamine structures represented by the above-mentioned general formula (1) (hereinafter often referred to as arylamine compound I) is a novel compound having higher degrees of hole injection property and mobility than those of the conventional hole-transporting materials, having excellent electron-blocking capability, a high degree of stability for the electrons and improved stability in the form of a thin film. The arylamine compound I is excellent in regard to its heat resistance, too. Therefore, the arylamine compound I is favorably used for forming various layers of the organic EL device of the present invention.

Concretely, the arylamine compound I is favorably used as a material for constituting the hole injection layer and/or the hole-transporting layer. The organic EL device is capable of confining the excitons formed in the luminous layer, permits the holes and the electrons to be recombined at an increased probability making it possible to attain a high luminous efficiency and, further, works on a decreased driving voltage contributing to lengthening the durability.

Besides, according to the present invention, the anthracene derivative represented by the above-mentioned general formula (2) (often referred to as anthracene derivative II) is favorably used as a material for constituting the electron-transporting layer. This is because the anthracene derivative II has excellent electron injecting·transporting capability and, further, has excellent stability and durability in the form of a thin film.

Further, according to the present invention, the anthracene derivative represented by the above-mentioned general formula (3) (often referred to as anthracene derivative III) is favorably used as a host material in the luminous layer and, specifically, as a host material in the luminous layer that contains a blue color-emitting dopant. This is because the anthracene derivative III has superior luminous efficiency to those of the conventional materials.

Moreover, the arylamine compound I has excellent electron-blocking capability as well as superior hole-transporting property to those of the conventional materials and, further, has a high stability in the form of a thin film. Therefore, the arylamine compound I is favorably used also as a material for constituting the electron-blocking layer. The organic EL device having the electron-blocking layer is capable of being driven on a low voltage yet realizing a high luminous efficiency, and features improved resistance against the electric current and improved maximum brightness.

Further, the arylamine compound I has superior hole-transporting capability to those of the conventional materials, and has a wide band gap. Therefore, the arylamine compound I is favorably used as a material for constituting the luminous layer and specifically, as a host material for carrying the dopant. The organic EL device having such a luminous layer drives on a low voltage and features improved luminous efficiency.

As described above, the present invention selects materials which are excellent in their hole injecting· transporting capability, electron injecting·transporting capability, stability in the form of a thin film and durability, and uses them in a suitable combination. Therefore, the organic EL device of the present invention is capable of efficiently injecting and transporting the holes from the hole-transporting layer into the luminous layer as compared to the conventional organic EL devices. The organic EL device of the invention, further, features improved electron-transporting efficiency from the electron-transporting layer into the luminous layer. As a result, there is realized an organic EL device that has a high efficiency, drives on a low voltage and features a long life.

### Brief Description of the Drawings:

[Fig. 1] It is a ¹H-NMR chart of a compound 33 of Synthesis Example 1.
[Fig. 2] It is a view illustrating the constitution of organic EL devices of Device Example 1 and Comparative Device Example 1.
[Fig. 3] It is a diagram showing structural formulas of compounds 1 to 10 that pertain to an arylamine compound I.
[Fig. 4] It is a diagram showing structural formulas of compounds 11 to 20 that pertain to the arylamine compound I.
[Fig. 5] It is a diagram showing structural formulas of compounds 21 to 30 that pertain to the arylamine compound I.
[Fig. 6] It is a diagram showing structural formulas of compounds 31 to 40 that pertain to the arylamine compound I.
[Fig. 7] It is a diagram showing structural formulas of compounds 41 to 48 that pertain to the arylamine compound I.
[Fig. 8] It is a diagram showing structural formulas of compounds 49 to 58 that pertain to the arylamine compound I.
[Fig. 9] It is a diagram showing structural formulas of compounds 59 to 65 that pertain to the arylamine compound I.
[Fig. 10] It is a diagram showing structural formulas of compounds 2a-1 to 2a-8 that pertain to an anthracene derivative II.
[Fig. 11] It is a diagram showing structural formulas of compounds 2a-9 to 2a-16 that pertain to the anthracene derivative II.
[Fig. 12] It is a diagram showing structural formulas of compounds 2a-17 to 2a-20 that pertain to the anthracene derivative II.
[Fig. 13] It is a diagram showing structural formulas of compounds 2b-1 to 2b-8 that pertain to the anthracene derivative II.
[Fig. 14] It is a diagram showing structural formulas of compounds 2b-9 to 2b-16 that pertain to the anthracene derivative II.
[Fig. 15] It is a diagram showing structural formulas of compounds 2c-1 to 2c-6 that pertain to the anthracene derivative II.
[Fig. 16] It is a diagram showing structural formulas of compounds 2c-7 to 2c-12 that pertain to the anthracene derivative II.
[Fig. 17] It is a diagram showing structural formulas of compounds 2c-13 to 2c-18 that pertain to the anthracene derivative II.
[Fig. 18] It is a diagram showing structural formulas of compounds 2c-19 to 2c-24 that pertain to the anthracene derivative II.
[Fig. 19] It is a diagram showing structural formulas of compounds 2c-25 to 2c-30 that pertain to the anthracene derivative II.
[Fig. 20] It is a diagram showing structural formulas of compounds 3-1 to 3-6 that pertain to an anthracene derivative III.
[Fig. 21] It is a diagram showing structural formulas of compounds 3-7 to 3-11 that pertain to the anthracene derivative III.

### Modes for Carrying Out the Invention:

The organic EL device of the present invention has a basic structure in which at least an anode, a hole-transporting layer, a luminous layer, an electron-transporting layer and a cathode are arranged in this order on a substrate.

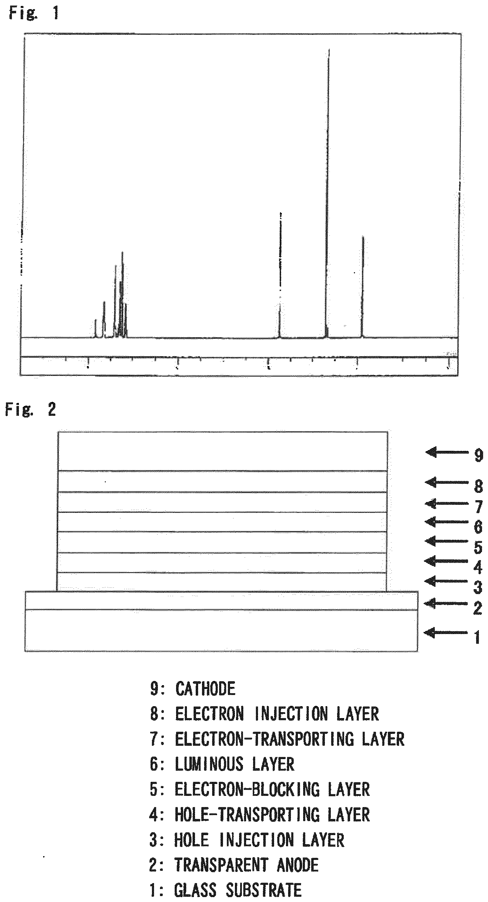

The organic EL device of the present invention can be realized in a variety of layer structures if it does not depart from the above-mentioned basic structure. For instance, it is allowable to provide a hole injection layer between the anode and the hole-transporting layer, to provide an electron-blocking layer between the hole-transporting layer and the luminous layer, to provide a hole-blocking layer between the luminous layer and the electron-transporting layer, or to provide an electron injection layer between the electron-transporting layer and the cathode. Moreover, some of the organic layers may be omitted or may be used to also serve as other layers. For instance, the organic layers can be used to also serve as the hole injection layer and as the hole-transporting layer, or to serve as the electron injection layer and as the electron-transporting layer. It is, further, allowable to laminate two or more organic layers that have the same function. For instance, two hole-transporting layers can be laminated one upon the other, two luminous layers can be laminated one upon the other, or two electron-transporting layers can be laminated one upon the other. Fig. 2 shows the constitution of layers employed in the EXAMPLES described later. Namely, Fig. 2 shows the constitution of layers in which a transparent anode 2, a hole injection layer 3, a hole-transporting layer 4, an electron-blocking layer 5, a luminous layer 6, an electron-transporting layer 7, an electron injection layer 8 and a cathode 9 are formed in this order on a glass substrate 1.

According to the present invention though the individual layers will be described later in detail, an important feature resides in that an arylamine compound I represented by the following general formula (1) is contained in at least one layer selected from the group consisting of the hole-transporting layer, luminous layer, electron-blocking layer that is formed as required, and hole injection layer that is formed as required. When the arylamine compound I is used in a plurality of layers that are neighboring each other, such plurality of layers should have different layer constitutions.

### <Arylamine compound I>

The arylamine compound I has a structure represented by the following general formula (1),

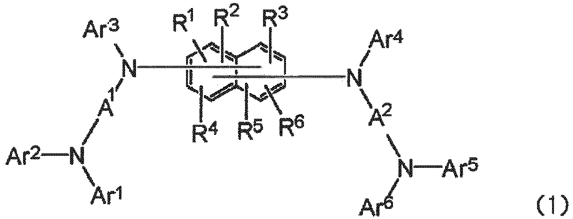

The arylamine compound I includes, for example, the following three embodiments depending on the positions to where R¹ to R⁶ are bonded on the naphthalene ring.

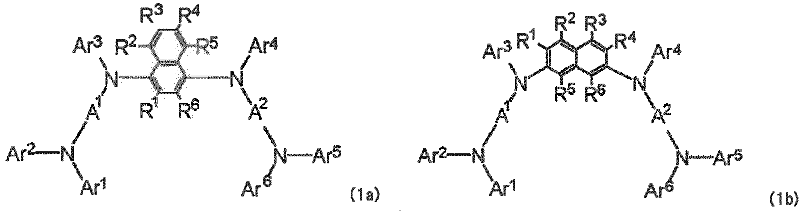

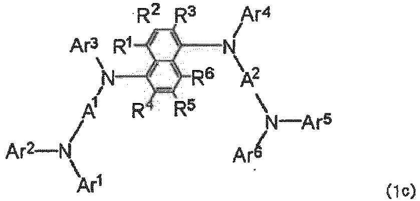

### (Ar¹ to Ar⁶)

Ar¹ to Ar⁶ may be the same or different, and are aromatic hydrocarbon groups, aromatic heterocyclic groups or condensed polycyclic aromatic groups. In this specification, the condensed polycyclic aromatic groups have no hetero atom (e.g., nitrogen atom, oxygen atom or sulfur atom) in the skeletons thereof.

As the aromatic hydrocarbon group, aromatic heterocyclic group or condensed polycyclic aromatic group represented by Ar¹ to Ar⁶, there can be concretely exemplified phenyl group, biphenylyl group, terphenylyl group, naphthyl group, anthracenyl group, phenanthrenyl group, fluorenyl group, indenyl group, pyrenyl group, perylenyl group, fluoranthenyl group, triphenylenyl group, pyridyl group, pyrimidinyl group, triazinyl group, furyl group, pyrrolyl group, thienyl group, quinolyl group, isoquinolyl group, benzofuranyl group, benzothienyl group, indolyl group, carbazolyl group, benzoxazolyl group, benzothiazolyl group, quinoxalinyl group, benzimidazolyl group, pyrazolyl group, dibenzofuranyl group, dibenzothienyl group, naphthyridinyl group, phenanthrolinyl group, acrydinyl group and carbolinyl group.

The aromatic hydrocarbon group, aromatic heterocyclic group or condensed polycyclic aromatic group represented by Ar¹ to Ar⁶ may be unsubstituted or may have a substituent. As the substituent, there can be exemplified the following groups in addition to the deuterium atom, cyano group and nitro group.
Halogen atoms such as fluorine atom, chlorine atom, bromine atom and iodine atom;
Alkyl groups having 1 to 6 carbon atoms, such as methyl group, ethyl group, n-propyl group, isopropyl group, n-butyl group, isobutyl group, tert-butyl group, n-pentyl group, isopentyl group, neopentyl group and n-hexyl group;
Alkyloxy groups having 1 to 6 carbon atoms, such as methyloxy group, ethyloxy group and propyloxy group;
Alkenyl groups, such as vinyl group and allyl group;
Aryloxy groups, such as phenyloxy group and tolyloxy group;
Arylalkyloxy groups such as benzyloxy group and phenetyloxy group;
Aromatic hydrocarbon groups or condensed polycyclic aromatic groups, such as phenyl group, biphenylyl group, terphenylyl group, naphthyl group, anthracenyl group, phenanthrenyl group, fluorenyl group, indenyl group, pyrenyl group, perylenyl group, fluoranthenyl group and triphenylenyl group;
aromatic heterocyclic groups, such as pyridyl group, pyrimidinyl group, triazinyl group, thienyl group, furyl group, pyrolyl group, quinolyl group, isoquinolyl group, benzofuranyl group benzothienyl group, indolyl group, carbazolyl group, benzoxazolyl group, benzothiazolyl group, quinoxalinyl group, benzimidazolyl group, pyrazolyl group, dibenzofuranyl group, dibenzothienyl group and carbolinyl group;
Arylvinyl groups, such as styryl group and naphthylvinyl group; and
Acyl groups, such as acetyl group and benzoyl group.

Here, the alkyl group having 1 to 6 carbon atoms, the alkyloxy group having 1 to 6 carbon atoms and the alkenyl group may be in the form of either straight chains or branched chains . The above substituent may not have been substituted or may have been substituted with the substituent described above. Further, the above substituents may be present independently from each other without forming any ring, or may be bonded to each other via a single bond, a substituted or unsubstituted methylene group, an oxygen atom or a sulfur atom to form a ring.

### (A¹, A²)

A¹ and A² may be the same or different, and are divalent aromatic hydrocarbon groups, divalent aromatic heterocyclic groups or divalent condensed polycyclic aromatic groups.

The divalent aromatic hydrocarbon group, divalent aromatic heterocyclic group or divalent condensed polycyclic aromatic group is a divalent group obtained by removing two hydrogen atoms from the aromatic hydrocarbon, aromatic heterocyclic compound or condensed polycyclic aromatic compound.

As the aromatic hydrocarbon group, aromatic heterocyclic group or condensed polycyclic aromatic group, there can be concretely exemplified benzene, biphenyl, terphenyl, tetrakisphenyl, styrene, naphthalene, anthracene, acenaphthalene, fluorene, phenanthrene, indane, pyrene, triphenylene, pyridine, pyrimidine, triazine, pyrrole, furan, thiophene, quinolone, isoquinolene, benzofurane, benzothiophene, indoline, carbazole, carboline, benzoxazole, benzothiazole, quinoxalene, benzimidazole, pyrazole, dibenzofurane, dibenzothiophene, naphthylidine, phenanthroline, acridine and acridan.

The divalent aromatic hydrocarbon group, divalent aromatic heterocyclic group or divalent condensed polycyclic aromatic group represented by A¹ and A² may be unsubstituted or may have a substituent. As the substituent, there can be exemplified those that were exemplified above as the substituents that may be possessed by the aromatic hydrocarbon group, aromatic heterocyclic group or condensed polycyclic aromatic group represented by Ar¹ to Ar⁶. The same also holds for the forms that can be assumed by the substituents.

### (R¹ to R⁶)

R¹ to R⁶ may be the same or different, and are hydrogen atoms, deuterium atoms, fluorine atoms, chlorine atoms, cyano groups, nitro groups, alkyl groups having 1 to 6 carbon atoms, cycloalkyl groups having 5 to 10 carbon atoms, alkenyl groups having 2 to 6 carbon atoms, alkyloxy groups having 1 to 6 carbon atoms, cycloalkyloxy groups having 5 to 10 carbon atoms, aromatic hydrocarbon groups, aromatic heterocyclic groups, condensed polycyclic aromatic groups or aryloxy groups. The alkyl group having 1 to 6 carbon atoms, alkenyl group having 2 to 6 carbon atoms and alkyloxy group having 1 to 6 carbon atoms may be in the form of either straight chains or branched chains.

These groups may be present independently from each other without forming any ring, or may be bonded to each other via a single bond, a substituted or unsubstituted methylene group, an oxygen atom or a sulfur atom to form a ring.

As the alkyl group having 1 to 6 carbon atoms, cycloalkyl group having 5 to 10 carbon atoms or alkenyl group having 2 to 6 carbon atoms represented by R¹ to R⁶, there can be concretely exemplified methyl group, ethyl group, n-propyl group, isopropyl group, n-butyl group, isobutyl group, tert-butyl group, n-pentyl group, isopentyl group, neopentyl group, n-hexyl group, cyclopentyl group, cyclohexyl group, 1-adamantyl group, 2-adamantyl group, vinyl group, allyl group, isopropenyl group and 2-butenyl group.

The alkyl group having 1 to 6 carbon atoms, cycloalkyl group having 5 to 10 carbon atoms or alkenyl group having 2 to 6 carbon atoms represented by R¹ to R⁶ may be unsubstituted or may have a substituent. As the substituent, there can be exemplified the following groups in addition to the deuterium atom, cyano group and nitro group.
Halogen atoms such as fluorine atom, chlorine atom, bromine atom and iodine atom;
Alkyloxy groups having 1 to 6 carbon atoms, such as methyloxy group, ethyloxy group and propyloxy group;
Alkenyl groups, such as vinyl group and allyl group;
Aryloxy groups, such as phenyloxy group and tolyloxy group;
Arylalkyloxy groups such as benzyloxy group and phenetyloxy group;
Aromatic hydrocarbon groups or condensed polycyclic aromatic groups, such as phenyl group, biphenylyl group, terphenylyl group, naphthyl group, anthracenyl group, phenanthrenyl group, fluorenyl group, indenyl group, pyrenyl group, perylenyl group, fluoranthenyl group and triphenylenyl group;
aromatic heterocyclic groups, such as pyridyl group, pyrimidinyl group, triazinyl group, thienyl group, furyl group, pyrolyl group, quinolyl group, isoquinolyl group, benzofuranyl group, benzothienyl group, indolyl group, carbazolyl group, benzoxazolyl group, benzothiazolyl group, quinoxalinyl group, benzimidazolyl group, pyrazolyl group, dibenzofuranyl group, dibenzothienyl group and carbolinyl group.

Here, the alkyloxy group having 1 to 6 carbon atoms and the alkenyl group may be in the form of either straight chains or branched chains. The above substituent may not be substituted or may be substituted with the substituent described above. Further, the above substituents may be present independently from each other without forming any ring, or may be bonded to each other via a single bond, a substituted or unsubstituted methylene group, an oxygen atom or a sulfur atom to form a ring.

As the alkyloxy group having 1 to 6 carbon atoms or the cycloalkyloxy group having 5 to 10 carbon atoms represented by R¹ to R⁶, there can be concretely exemplified methyloxy group, ethyloxy group, n-propyloxy group, isopropyloxy group, n-butyloxy group, tert-butyloxy group, n-pentyloxy group, n-hexyloxy group, cyclopentyloxy group, cyclohexyloxy group, cycloheptyloxy group, cyclooctyloxy group, 1-adamantyloxy group and 2-adamantyloxy group.

These groups may be unsubstituted or may have a substituent. As the substituent, there can be exemplified those that were exemplified above as the substituents that may be possessed by the alkyl group having 1 to 6 carbon atoms, cycloalkyl group having 5 to 10 carbon atoms or alkenyl groups having 2 to 6 carbon atoms represented by R¹ to R⁶ mentioned above. The same also holds for the forms that can be assumed by the substituents.

As the aromatic hydrocarbon group, aromatic heterocyclic group or condensed polycyclic aromatic group represented by R¹ to R⁶, there can be exemplified those that were described above as the aromatic hydrocarbon group, aromatic heterocyclic group or condensed polycyclic aromatic group represented by Ar¹ to Ar⁶.

These groups may be unsubstituted or may have a substituent. As the substituent, there can be exemplified those that were exemplified above as the substituents that may be possessed by the aromatic hydrocarbon group, aromatic heterocyclic group or condensed polycyclic aromatic group represented by Ar¹ to Ar⁶. The same also holds for the forms that can be assumed by the substituents.

As the aryloxy group represented by R¹ to R⁶, there can be concretely exemplified phenyloxy group, biphenylyloxy group, terphenylyloxy group, napthyloxy group, anthracenyloxy group, phenanthrenyloxy group fluorenyloxy group, indenyloxy group, pyrenyloxy group and perylenyloxy group.

The aryloxy group represented by R¹ to R⁶ may be unsubstituted or may have a substituent. As the substituent, there can be exemplified those that were exemplified above as the substituents that may be possessed by the aromatic hydrocarbon group, aromatic heterocyclic group or condensed polycyclic aromatic group represented by Ar¹ to Ar⁶. The same also holds for the forms that can be assumed by the substituents.

### (Preferred embodiments of the arylamine compound I)

Preferred embodiments of the arylamine compound I will now be described. In the description of the preferred embodiments, the groups that have not been specified to be substituted/unsubstituted may or may not have a substituent.

The arylamine compound I is represented, preferably, by the above-mentioned general formula (1a), (1b) or (1c) and, more preferably, by the above-mentioned general formula (1b).

It is, further, desired that the arylamine compound I has symmetry.

Ar¹ to Ar⁶ may be the same or different, and are, preferably, aromatic hydrocarbon groups, condensed polycyclic aromatic groups, benzofuranyl groups, benzothienyl groups, carbazolyl groups, dibenzofurayl groups or dibenzothienyl groups and, concretely, are phenyl groups, biphenylyl groups, naphthyl groups, anthracenyl groups, phenanthrenyl groups, fluorenyl groups, benzofuranyl groups, benzothienyl groups, carbazolyl groups, dibenzofuranyl groups or dibenzothienyl groups. Specifically, they are aromatic hydrocarbon groups or condensed polycyclic aromatic groups and, most preferably, are phenyl groups, biphenylyl grops or naphthyl groups. These groups may have a substituent but, more preferably, are unsubstituted.

A¹ and A² maybe the same or different, and are, preferably, divalent groups obtained by removing two hydrogen atoms from the aromatic hydrocarbon group, condensed polycyclic aromatic group, benzofuran, benzothiophene, carbazole, dibenzofuran or dibenzothiophene and, more preferably, are divalent groups obtained by removing two hydrogen atoms from the aromatic hydrocarbon group or the condensed polycyclic aromatic group. Concretely, they are divalent groups obtained by removing two hydrogen atoms from benzene, biphenyl, naphthalene, anthracene, fluorene, phenanthrene, benzofuran, benzothiophene, carbazole, dibenzofuran or dibenzothiophene and, more preferably, are divalent groups obtained by removing two hydrogen atoms from benzene, biphenyl or naphthalene.

R¹ to R⁶ may be the same or different and are, preferably, hydrogen atoms, deuterium atoms, aromatic hydrocarbon groups, condensed polycyclic aromatic groups, benzofuranyl groups, benzothienyl groups, carbazolyl groups, dibenzofuranyl groups or dibenzothienyl groups. The aromatic hydrocarbon groups, condensed polycyclic aromatic groups, benzofuranyl groups, benzothienyl groups, carbazolyl groups, dibenzofuranyl groups or dibenzothienyl groups may have a substituent but, more preferably, are unsubstituted. More preferably, they are hydrogen atoms, deuterium atoms, phenyl groups, biphenyl groups, naphthyl groups, anthracenyl groups, phenanthrenyl groups, fluorenyl groups, benzofuranyl groups, benzothienyl groups, carbazolyl groups, dibenzofuranyl groups or dibenzothienyl groups. Specifically preferably, they are hydrogen atoms, deuterium atoms, phenyl groups or naphthyl groups.

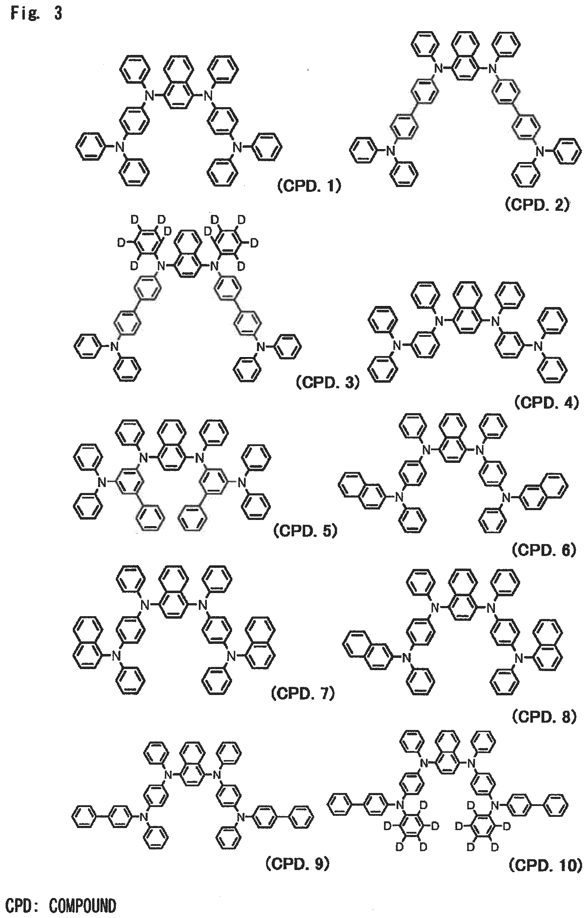

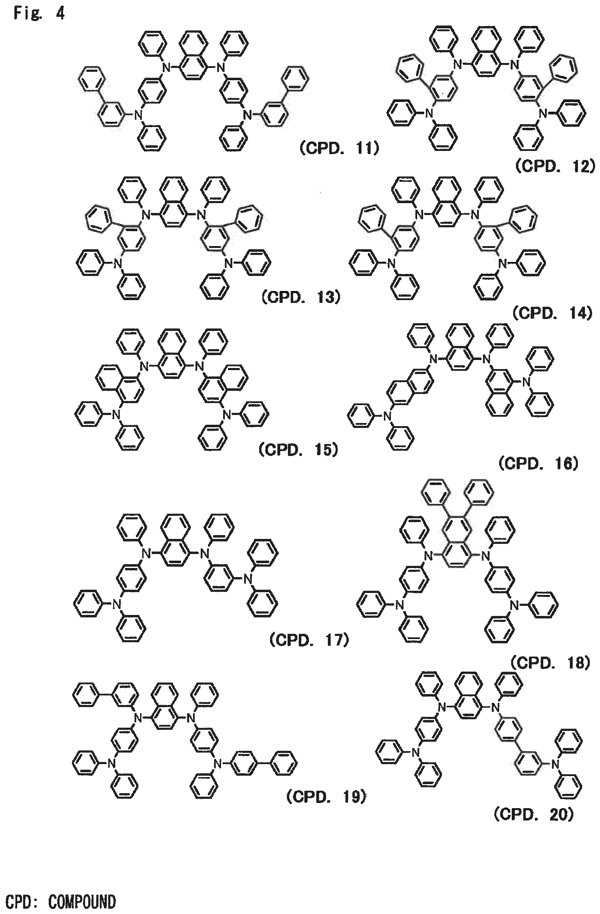

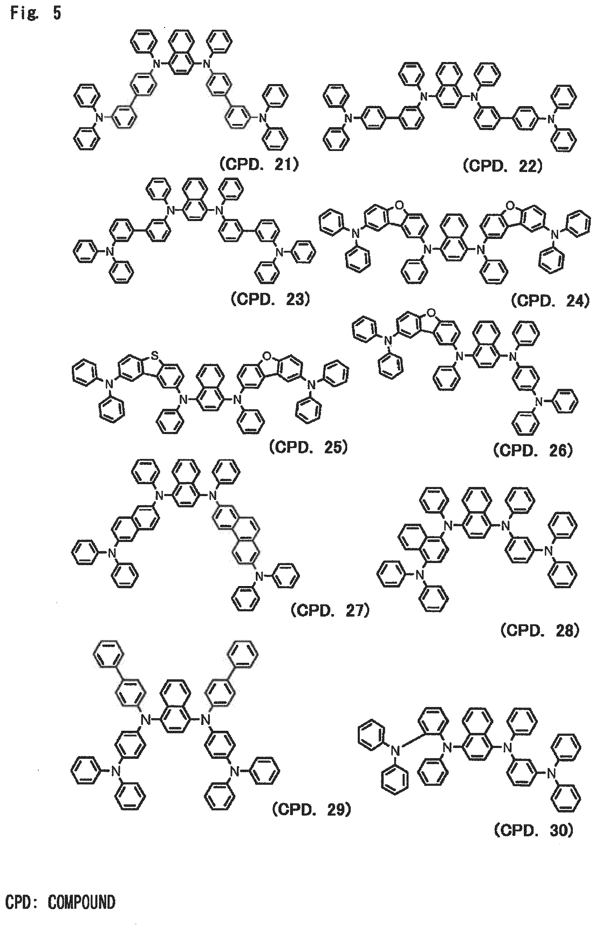

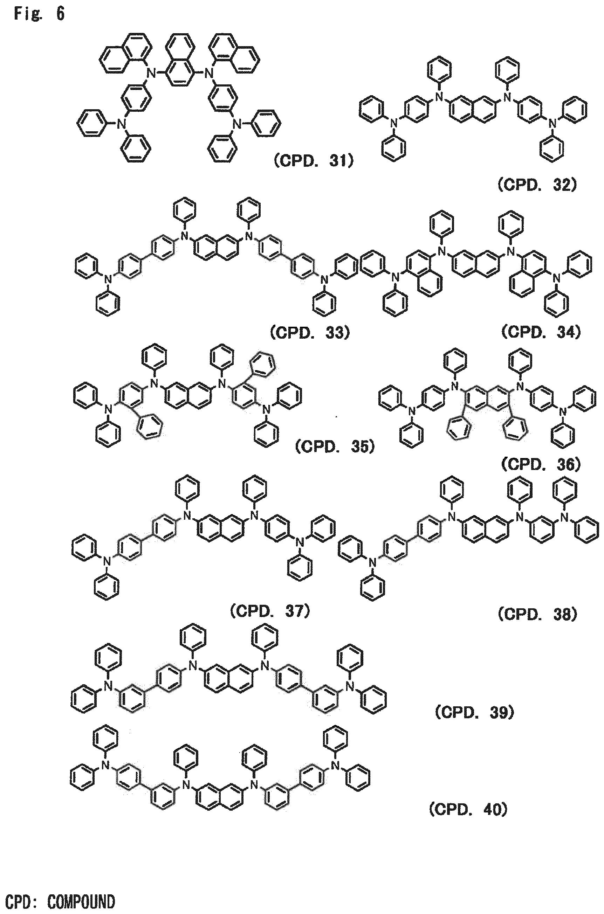

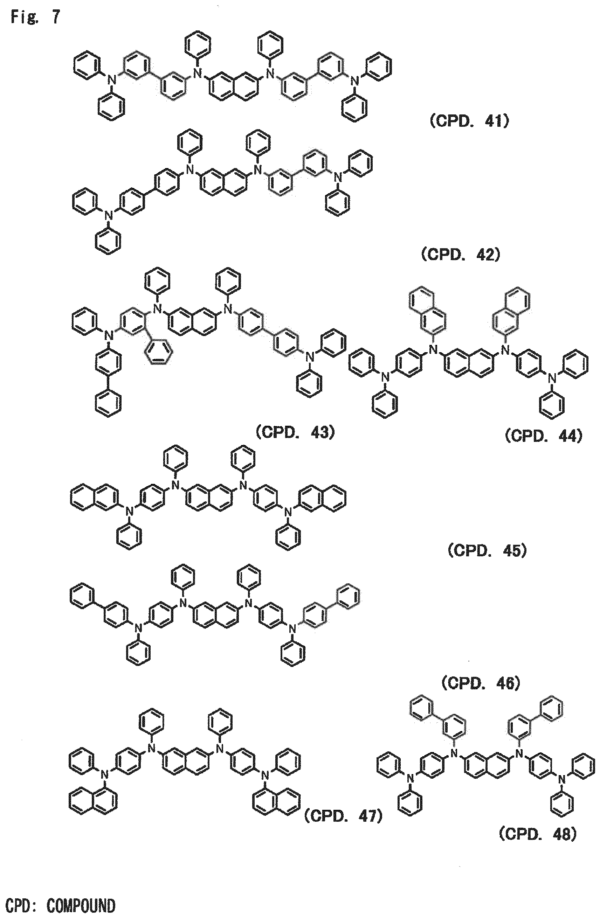

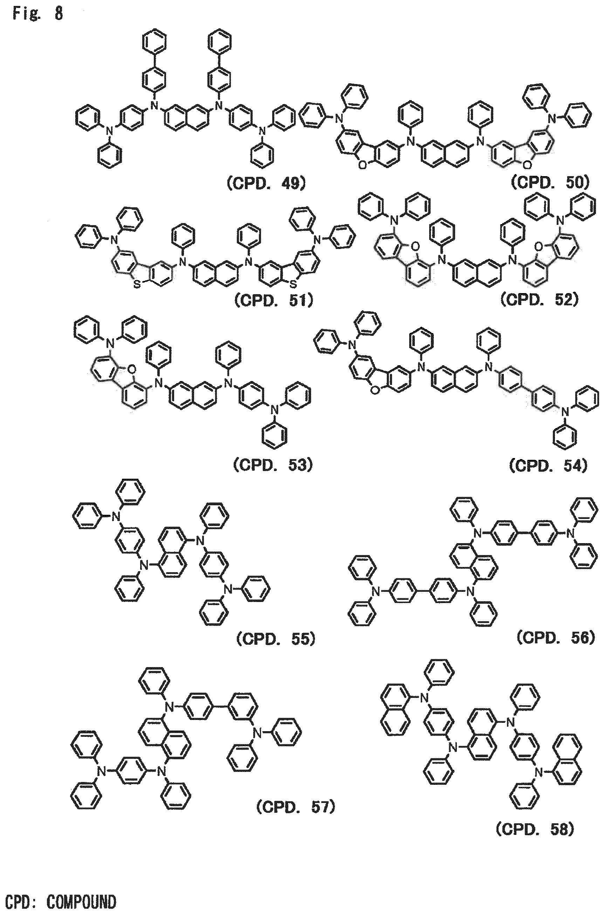

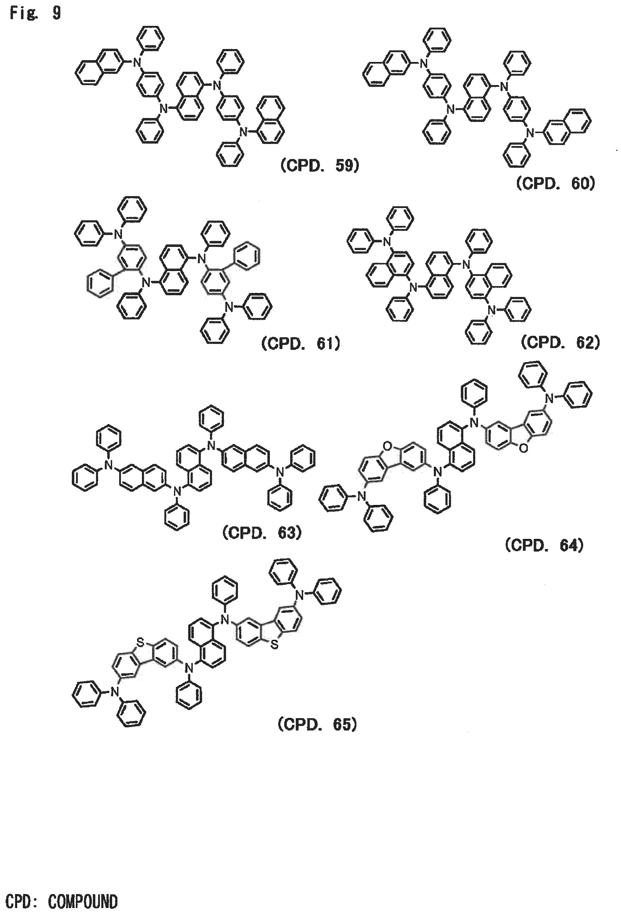

Fig. 3 to 9 show concrete examples of preferred forms of the arylamine compound I, which, however, are in no way limited to these forms only. In these concrete examples, the compounds 1 to 31 can be represented by the above general formula (1a). The compounds 32 to 54 can be represented by the above general formula (1b) while the compounds 55 to 65 can be represented by the above general formula (1c). D stands for a deuterium atom.

The arylamine compound I can be prepared by a known method such as Buchwald-Hartwig coupling.

The arylamine compound I can be refined by column chromatography, by the adsorption refining method using silica gel, activated carbon or activated clay, by the recrystallization method or the crystallization method using a solvent or by the sublimation method. The compounds are identified by the NMR analysis. As for properties, a glass transition point (Tg) and a work function can be measured.

The glass transition point (Tg) serves as an index of stability in the form of a thin film. The glass transition point (Tg) is measured by using a powder thereof and a high-sensitivity differential scanning calorimeter (DSC 3100S manufactured by Bruker AXS K.K.).

The work function serves as an index for transporting the holes. The work function can be measured by forming a film which is as thin as 100nm on an ITO substrate and by using an ionization potential-measuring instrument (Model PYS-202, manufactured by Sumitomo Heavy Industries, Ltd.).

In addition to the arylamine compound I, the compounds (e.g., anthracene derivatives II, III described later) used for the organic EL device of the invention, too, can be refined after they have been synthesized and measured for their properties by the same methods.

In the organic EL device of the present invention, the layers can assume various forms so far as the above-mentioned arylamine compound I is used. The layers will now be described in detail with reference to Fig. 2.

### <Anode 2>

In the organic EL device of the present invention, an anode 2 is provided on a glass substrate 1. As the anode 2, there is used an electrode material having a large work function, such as ITO or gold.

### <Hole injection layer 3>

A hole injection layer 3 can be provided between the anode 2 and a hole-transporting layer 4. As the hole injection layer 3, the arylamine compound I is preferably used. It is also allowable to use any other known materials.

As the known materials, there can be used porphyline compounds as represented by copper phthalocyanine; materials, for example, triphenylamine derivatives of the star burst type and various triphenylamine tetramers; acceptor-type heterocyclic compounds such as hexacyanoazatriphenylene; and a high molecular materials of the application type.

In addition to using the materials that are usually used for forming the hole injection layer, it is also allowable to use the materials P-doped with a trisbromophenylaminehexachloroantimony or a Radialene derivative (see, for example, International Laid-Open WO2014/009310), or to use a high-molecular compound having a benzidine derivative such as TPD as part of its structure.

The hole injection layer 3 can be obtained by forming a thin film of the above materials relying on a known method such as the vacuum evaporation method, the spin-coating method or the ink-jet method. The layers described below, too, can similarly be obtained by forming thin films by the known method such as the vacuum evaporation method, the spin-coating method or the ink-jet method.

### <Hole-transporting layer 4>

The hole-transporting layer 4 is provided on the anode 2 (or the hole injection layer 3). As the hole-transporting layer 4, it is desired to use the above arylamine compound I. As the hole-transporting layer 4, there can be also used the known hole-transporting materials that are described below so far as they do not impair the effects of the present invention. Benzidine derivatives such as,
NPD,
N,N'-Diphenyl-N,N'-di(m-tolyl) benzidine (TPD);
N,N,N' ,N'-Tetrabiphenylylbenzidine;
1,1-Bis[4-(di-4-tolylamino)phenyl] cyclohexane; and Various triphenylamine trimers and tetramers.

These materials can be used in a single kind to form the film (single film) but can also be used being mixed with other materials to form the film (mixed film) . The film can similarly be formed for the organic layers, too, that are described below.

The hole-transporting layer 4 may have a structure in which the layers of single films are laminated one upon the other, a structure in which the layers of mixed films are laminated one upon the other, or a structure in which the layers of single films and the layers of mixed films are laminated one upon the other. The organic layers described below can also assume the same structures.

When there is provided a layer that serves both as the hole injection layer 3 and the hole-transporting layer 4, there can be used the above arylamine compound I or a high-molecular material of the application type, such as poly(3,4-ethylenedioxythiophene) (PEDOT)/poly(styrene sulfonate) (PSS).

In addition to using the materials that are usually used for forming the hole-transporting layer, it is also allowable to use the materials P-doped with a trisbromophenylaminehexachloroantimony or a Radialene derivative (see, for example, International Laid-Open WO2014/009310), or to use a high-molecular compound having a benzidine derivative such as TPD as part of its structure.

### <Electron-blocking layer 5>

An electron-blocking layer 5 can be provided between the hole-transporting layer 4 and a luminous layer 6. As the electron-blocking layer 5, the above arylamine compound I can be preferably used. The electron-blocking layer 5 can contain known compounds having electron-blocking capability that are described below so far as they do not impair the effects of the present invention.
Carbazole derivatives such as,
4,4',4"-Tri(N-carbazolyl)triphenylamine (TCTA);
9,9-Bis [4-(carbazole-9-il)phenyl] fluorene;
1,3-Bis(carbazole-9-il)benzene (mCP);
2,2-Bis(4-carbazole-9-ilphenyl)adamantane (Ad-Cz);
Triarylamine compound having a triphenylsilyl group, such as 9-[4-(carbazole-9-il)phenyl]-9-[4-(triphenylsilyl) phenyl]-9H-fluorene.

### <Luminous layer 6>

The luminous layer 6 is formed on the hole-transporting layer 4 (or the electron-blocking layer 5). As the luminous layer 6, there can be used metal complexes of quinolynol derivatives such as Alq₃, various kinds of other metal complexes, anthracene derivatives, bisstyrylbenzene derivatives, pyrene derivatives, oxazole derivatives, and polyparaphenylenevinylene derivatives.

It is also allowable to constitute the luminous layer by using a host material and a dopant material. As the host material, there can be used the above-mentioned luminous materials. There can be further preferably used the above arylamine compound I or the anthracene derivative.

As the anthracene derivative, an anthracene derivative III represented by the following general formula (3) is preferred. The anthracene derivative III will now be described.

### Anthracene derivative III;

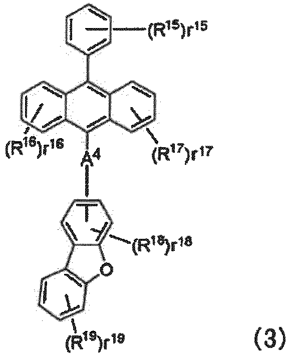

### (R¹⁵ to R¹⁹)

R¹⁵ to R¹⁹ may be the same or different, and are deuterium atoms, alkyl groups having 1 to 30 carbon atoms, alkenyl groups having 2 to 30 carbon atoms, alkynyl groups having 2 to 30 carbon atoms, cycloalkyl groups having 3 to 30 carbon atoms, cycloalkenyl groups having 5 to 30 carbon atoms, alkyloxy groups having 1 to 30 carbon atoms, aryloxy groups having 6 to 30 carbon atoms, alkylthio groups having 1 to 30 carbon atoms, arylthio groups having 5 to 30 carbon atoms, alkylamino groups having 1 to 30 carbon atom, arylamino groups having 5 to 30 carbon atoms, aryl groups having 6 to 50 carbon atoms, aromatic heterocyclic groups having 2 to 50 carbon atoms, cyano groups, nitro groups, halogen atoms, amino groups, hydroxy groups or groups -CO-R²⁰, and R²⁰ is a hydrogen atom, a hydroxy group, an alkyloxy group having 1 to 6 carbon atoms or an aryloxy group having 6 to 30 carbon atoms. The alkyl group having 1 to 30 carbon atoms, alkenyl group having 2 to 30 carbon atoms and alkynyl group having 2 to 30 carbon atoms may be in the form of either straight chains or branched chains.

These groups may be present independently from each other without forming any ring but may be bonded to each other via a single bond, a substituted or unsubstituted methylene group, an oxygen atom or a sulfur atom to form a ring.

As the alkyl group having 1 to 30 carbon atoms, alkenyl group having 2 to 30 carbon atoms, alkynyl group having 2 to 30 carbon atoms, cycloalkyl group having 3 to 30 carbon atoms or cycloalkenyl group having 5 to 30 carbon atoms represented by R¹⁵ to R¹⁹, there can be concretely exemplified methyl group, ethyl group, n-propyl group, isopropyl group, n-butyl group, isobutyl group, tert-butyl group, n-pentyl group, isopentyl group, neopentyl group, n-hexyl group, cyclopentyl group, cyclohexyl group, 1-adamantyl group, 2-adamantyl group, vinyl group, allyl group, isopropenyl group, 2-butenyl group, cyclopentenyl group, cyclohexenyl group, ethynyl group, isopropynyl group and 2-butynyl group.

The alkyl group having 1 to 30 carbon atoms, alkenyl group having 2 to 30 carbon atoms, alkynyl group having 2 to 30 carbon atoms, cycloalkyl group having 3 to 30 carbon atoms or cycloalkenyl group having 5 to 30 carbon atoms represented by R¹⁵ to R¹⁹, may be unsubstituted or may have a substituent. As the substituent, there can be exemplified the following groups in addition to the deuterium atom, cyano group and nitro group.
Halogen atoms such as fluorine atom, chlorine atom, bromine atom and iodine atom;
Alkyloxy groups having 1 to 6 carbon atoms, such as methyloxy group, ethyloxy group and propyloxy group;
Alkenyl groups, such as vinyl group and allyl group;
Aryloxy groups, such as phenyloxy group and tolyloxy group;
Arylalkyloxy groups such as benzyloxy group and phenetyloxy group;
Aromatic hydrocarbon groups or condensed polycyclic aromatic groups, such as phenyl group, biphenylyl group, terphenylyl group, naphthyl group, anthracenyl group, phenanthrenyl group, fluorenyl group, indenyl group, pyrenyl group, perylenyl group, fluoranthenyl group and triphenylenyl group;
aromatic heterocyclic groups, such as pyridyl group, pyrimidinyl group, triazinyl group, thienyl group, furyl group, pyrolyl group, quinolyl group, isoquinolyl group, benzofuranyl group, benzothienyl group, indolyl group, carbazolyl group, benzoxazolyl group, benzothiazolyl group, quinoxalinyl group, benzimidazolyl group, pyrazolyl group, dibenzofuranyl group, dibenzothienyl group and carbolinyl group.

Here, the alkyloxy group having 1 to 6 carbon atoms and the alkenyl group may be in the form of either straight chains or branched chains. The above substituent may not have been substituted or may have been substituted with the substituent described above. Further, the above substituents may be present independently from each other without forming any ring, or may be bonded to each other via a single bond, a substituted or unsubstituted methylene group, an oxygen atom or a sulfur atom to form a ring.

As the alkyloxy group having 1 to 30 carbon atoms, aryloxy group having 6 to 30 carbon atoms, alkylthio group having 1 to 30 carbon atoms, arylthio group having 5 t 30 carbon atoms, alkylamino group having 1 to 30 carbon atoms or arylamino group having 5 to 30 carbon atoms represented by R¹⁵ to R¹⁹, there can be concretely exemplified methyloxy group, ethyloxy group, n-propyloxy group, isopropyloxy group, n-butyloxy group, tert-butyloxy group, n-pentyloxy group, n-hexyloxy group, phenyloxy group, naphthyloxy group, methylthio group, ethylthio group, phenylthio group, naphthylthio group, dimethylamino group, diethylamino group, diphenylamino group and dinaphthylamino group.

The alkyloxy group having 1 to 30 carbon atoms, aryloxy group having 6 to 30 carbon atoms, alkylthio groups having 1 to 30 carbon atoms, arylthio group having 5 to 30 carbon atoms, alkylamino group having 1 to 30 carbon atoms or arylamino group having 5 to 30 carbon atoms represented by R¹⁵ to R¹⁹, may be unsubstituted or may have a substituent. As the substituent, there can be exemplified those that were exemplified above as the substituents that may be possessed by the alkyl group having 1 to 30 carbon atoms, alkenyl group having 2 to 30 carbon atoms, alkynyl group having 2 to 30 carbon atoms, cycloalkyl group having 3 to 30 carbon atoms or cycloalkenyl group having 5 to 30 carbon atoms represented by R¹⁵ to R¹⁹. The same also holds for the forms that can be assumed by the substituents.

As the aryl group having 6 to 50 carbon atoms or the aromatic heterocyclic group having 2 to 50 carbon atoms represented by R¹⁵ to R¹⁹, there can be concretely exemplified phenyl group, biphenylyl group, naphthyl group, anthracenyl group, phenanthrenyl group, fluorenyl group, benzofuranyl group, benzothienyl group, carbazolyl group, dibenzofuranyl group and dibenzothienyl group.

The aryl group having 6 to 50 carbon atoms or the aromatic heterocyclic group having 2 to 50 carbon atoms represented by R¹⁵ to R¹⁹, may be unsubstituted or may have a substituent. As the substituent, there can be exemplified those that were exemplified above as the substituents that may be possessed by the alkyl group having 1 to 30 carbon atoms, alkenyl group having 2 to 30 carbon atoms, alkynyl group having 2 to 30 carbon atoms, cycloalkyl group having 3 to 30 carbon atoms or cycloalkenyl group having 5 to 30 carbo atoms represented by R¹⁵ to R¹⁹. The same also holds for the forms that can be assumed by the substituents.

### (R²⁰)

As the alkyloxy group having 1 to 6 carbon atoms or the aryloxy group having 6 to 30 carbon atoms represented by R²⁰, there can be exemplified methyloxy group, ethyloxy group, propyloxy group, phenyloxy group, biphenylyloxy group, naphthyloxy group, anthracenyloxy group and phenanthrenyloxy group.

These groups may be unsubstituted or may have a substituent. As the substituent, there can be exemplified those that were exemplified above as the substituents that may be possessed by the alkyl group having 1 to 30 carbon atoms, alkenyl group having 2 to 30 carbon atoms, alkynyl group having 2 to 30 carbon atoms, cycloalkyl group having 3 to 30 carbon atoms or cycloalkenyl group having 5 to 30 carbo atoms represented by R¹⁵ to R¹⁹. The same also holds for the forms that can be assumed by the substituents.

### (r¹⁵ to r¹⁹)

Symbol r¹⁵ is an integer of 0 to 5 while r¹⁶, r¹⁷ and r¹⁹ are integers of 0 to 4, and r¹⁸ is an integer of 0 to 3. The case where r¹⁵ to r¹⁹ are 0 means that none of R¹⁵ to R¹⁹ are present, i.e., the benzene ring has not been substituted with the group represented by R¹⁵ to R¹⁹.

When r¹⁵ to r¹⁹ are integers other than 0 or 1 within the above-mentioned range, it means that a plurality of R¹⁵ to R¹⁹ are bonded to the same benzene ring. In this case, the plurality of groups that are bonded may be the same or different. Further, the groups may be present independently from each other without forming any ring or may be bonded to each other via a single bond, a substituted or unsubstituted methylene group, an oxygen atom or a sulfur atom to form a ring.

### (A⁴)

Symbol A⁴ represents a divalent aromatic hydrocarbon group, a divalent condensed polycyclic aromatic group or a single bond.

The divalent aromatic hydrocarbon group or the divalent condensed polycyclic aromatic group is a divalent group obtained by removing two hydrogen atoms from the aromatic hydrocarbon or the condensed polycyclic aromatic compound.

As the aromatic hydrocarbon group or the condensed polycyclic aromatic group, there can be concretely exemplified benzene, biphenyl, terphenyl, tetrakisphenyl, styrene, naphthalene, anthracene, acenaphthalene, fluorene, phenanthrene, indane, pyrene and triphenylene.

The divalent aromatic hydrocarbon group or the divalent condensed polycyclic aromatic group represented by A⁴ may be unsubstituted or may have a substituent. As the substituent, there can be exemplified those that were exemplified above as the substituents that may be possessed by the aromatic hydrocarbon group, aromatic heterocyclic group or condensed polycyclic aromatic group represented by Ar¹ to Ar⁶ in the above-mentioned general formula (1). The same also holds for the forms that can be assumed by the substituents.

### (Preferred embodiments of the anthracene derivative III)

Described below are preferred embodiments of the anthracene derivative III. In the description of the preferred embodiments, the groups that have not been specified to be substituted/unsubstituted may have a substituent or may not be substituted.

The anthracene derivative III is, preferably, represented by the following general formula (3a) or (3b) and is, more preferably, represented by the following general formula (3a).

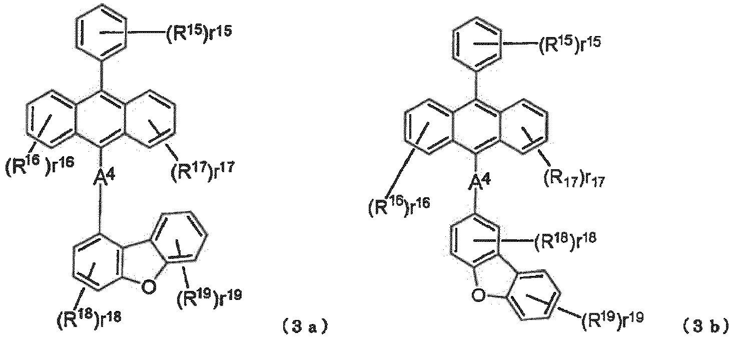

The groups R¹⁵ to R¹⁹ may be the same or different, and are, preferably, deuterium atoms, aryl groups having 6 to 50 carbon atoms, benzofuranyl groups, benzothienyl groups, carbazolyl groups, dibenzofuranyl groups or dibenzothienyl groups, more preferably, are deuterium atoms, phenyl groups, biphenylyl groups, naphthyl groups, anthracenyl groups, phenanthrenyl groups, fluorenyl groups, benzofuranyl groups, benzothienyl groups, carbazolyl groups, dibenzofuranyl groups or dibenzothienyl groups and, specifically, preferably, are deuterium atoms, phenyl groups, carbazolyl groups or dibenzofuranyl groups.

As r¹⁵, 0 or 5 is preferred, and 0 is more preferred. As r¹⁶ and r¹⁷, 0 is preferred. As r¹⁸, 0, 1 or 3 is preferred, and 3 is more preferred. As r¹⁹, 0 or 1 is preferred, and 0 is more preferred.

A⁴ is, preferably, a single bond or a divalent group obtained by removing two hydrogen atoms from benzene, biphenyl, naphthalene, anthracene, fluorine or phenanthrene. More preferably, A⁴ is a single bond or a divalent group obtained by removing two hydrogen atoms from benzene or naphthalene.

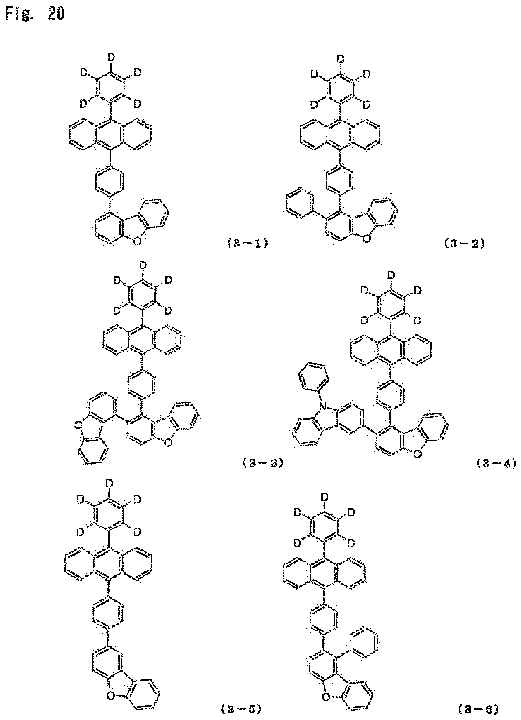

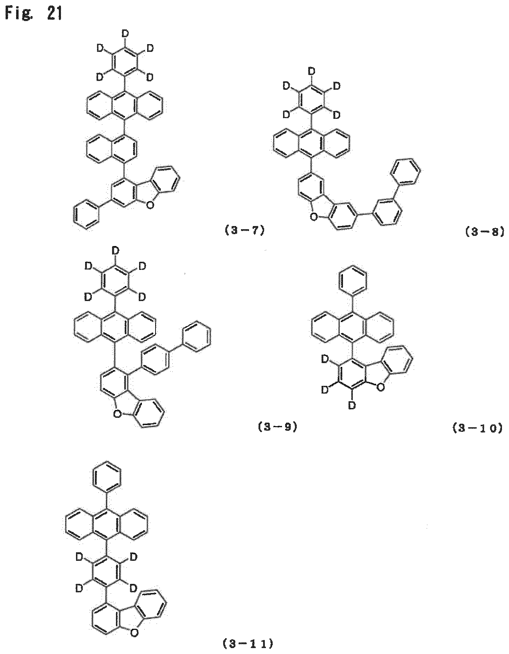

Figs. 20 and 21 show preferred concrete examples of the anthracene derivative III, to which only, however, the anthracene derivative III is in no way limited. In these concrete examples, the compounds 3-1 to 3-4, 3-7, 3-10 and 3-11 can be represented by the above general formula (3a) while the compounds 3-5, 3-6, 3-8 and 3-9 can be represented by the above general formula (3b). D represents a deuterium atom.

As the host material, there may be used a heterocyclic compound having an indole ring as a part of the structure, a heterocyclic compound having a carbazole ring as a part of the structure, a carbazole derivative, a thiazole derivative, a benzimidazole derivative or a polydialkylfluorene derivative.

As the dopant material, there can be, preferably, used a blue color-emitting dopant such as pyrene derivatives, as well as amine derivatives having a fluorine ring as a part of the structure; quinacridone, cumalin, rubrene, perylene, pyrene and derivatives thereof; benzopyran derivatives; indenophenanthrene derivatives; Rhodamine derivatives; and aminostyryl derivatives.

As the luminous material, it is also allowable to use a phosphorescent luminous material. As the phosphorescent luminous material, there can be used a phosphorescent luminous body of a metal complex such as of iridium or platinum. Concretely, there can be used a green luminous phosphor such as Ir (ppy)₃, a blue luminous phosphor such as Flrpic or Flr₆, and a red luminous phosphor such as Btp₂Ir(acac).

As the host material, in this case, there can be used the following hole injection·transporting host materials:
Carbazole derivatives such as 4,4'-di(N-carbazolyl) biphenyl (CBP), TCTA, mCP, etc.; and
Arylamine compounds such as the arylamine compound I described above.

It is, further, allowable to use the following electron-transporting host materials:
p-Bis(triphenylsilyl)benzene (UGH2), 2,2',2"-(1,3,5-phenylene)-tris(1-phenyl-1H-benzimidazole) (TPBl), etc.
Organic EL devices of high performance can be fabricated by using the above host materials.

To avoid the concentration quenching, the host material is desirably doped with the phosphorescent luminous material in an amount in a range of 1 to 30% by weight relative to the whole luminous layer relying on the vacuum coevaporation.

As the luminous material, further, it is also allowable to use a material that emits retarded fluorescence, such as CDCB derivative like PIC-TRZ, CC2TA, PXZ-TRZ or 4CzIPN.

### <Hole-blocking layer>

A hole-blocking layer (not shown) can be formed on the luminous layer 6. As the hole-blocking layer, there can be used a known compound having a hole-blocking action. As the known compound having the hole-blocking action, there can be exemplified phenanthrolene derivatives such as bathocuproin (BCP), metal complexes of quinolynol derivatives such as aluminum(III)bis (2-methyl-8- quinolinato) -4-phenyl phenolate (BAlq); various rare earth complexes; triazole derivatives; triazine derivatives; and oxadiazole derivatives. These materials may also be used as the materials for forming the electron-transporting layer.

### <Electron-transporting layer 7>

The electron-transporting layer 7 is provided on the luminous layer 6 (or the hole-blocking layer). As the electron-transporting layer 7, it is desired to use an anthracene derivative II represented by the following general formula (2).

### Anthracene derivative II;

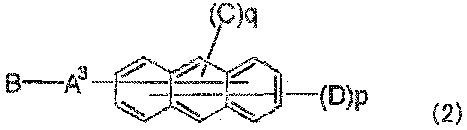

The anthracene derivative II includes, for example, the following three embodiments.

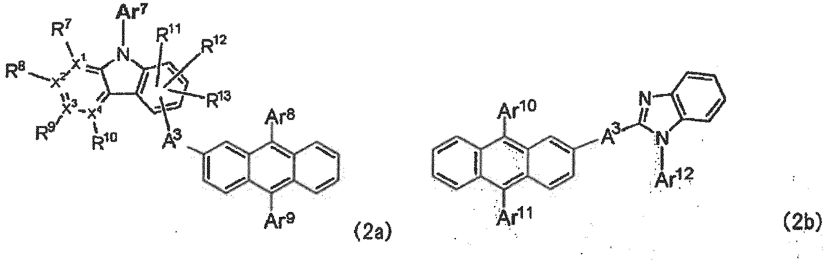

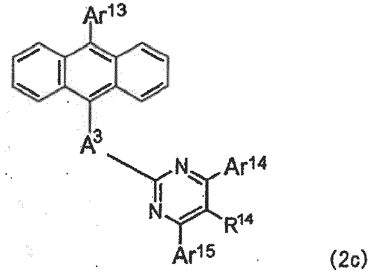

### (A³)

A³ represents a divalent aromatic hydrocarbon group, a divalent aromatic heterocyclic group, a divalent condensed polycyclic aromatic group or a single bond.

As the divalent aromatic hydrocarbon group, divalent aromatic heterocyclic group or divalent condensed polycyclic aromatic group represented by A³, there can be exemplified those which are the same as the divalent aromatic hydrocarbon groups, divalent aromatic heterocyclic groups or divalent condensed polycyclic aromatic groups represented by A¹ and A² in the above-mentioned general formula (1).

The divalent groups may not be substituted or may have a substituent. As the substituents, there can be exemplified those that were exemplified above as substituents that may be possessed by the aromatic hydrocarbon groups, aromatic heterocyclic groups or condensed polycyclic aromatic groups represented by Ar¹ to Ar⁶ in the above formula (1). The same also holds for the forms that can be assumed by the substituents.

### (B)

B represents an aromatic heterocyclic group. Concretely, there can be exemplified triazinyl group, pyridyl group, pyrimidinyl group, furyl group, pyrrolyl group, thienyl group, quinolyl group, isoquinolyl group, benzofuranyl group, benzothienyl group, indolyl group, carbazolyl group, benzoxazolyl group, benzothiazolyl group, quinoxalinyl group, benzoimidazolyl group, pyrazolyl group, dibenzofuranyl group, dibenzothienyl group, naphthyridinyl group, phenanthrolinyl group, acridinyl group and carbolinyl group.

The aromatic heterocyclic group represented by B may be unsubstituted but may have a substituent. As the substituent, there can be exemplified the following groups in addition to the deuterium atom, cyano group and nitro group.
Halogen atoms such as fluorine atom, chlorine atom, bromine atom and iodine atom;
Alkyl groups having 1 to 6 carbon atoms, such as methyl group, ethyl group, n-propyl group, isopropyl group, n-butyl group, isobutyl group, tert-butyl group, n-pentyl group, isopentyl group, neopentyl group and n-hexyl group;
Cycloalkyl groups having 5 to 10 carbon atoms, such as cyclopentyl group, cyclohexyl group, 1-adamantyl group and 2-adamantyl group;
Alkyloxy groups having 1 to 6 carbon atoms, such as methyloxy group, ethyloxy group and propyloxy group;
Cycloalkyloxy groups having 5 to 10 carbon atoms, such as cyclopentyloxy group, cyclohexyloxy group, 1-adamantyloxy group and 2-adamantyloxy group;
Alkenyl groups, such as vinyl group and allyl group;
Aryloxy groups, such as phenyloxy group and tolyloxy group;
Arylalkyloxy groups such as benzyloxy group and phenetyloxy group;
Aromatic hydrocarbon groups or condensed polycyclic aromatic groups, such as phenyl group, biphenylyl group, terphenylyl group, naphthyl group, anthracenyl group, phenanthrenyl group, fluorenyl group, indenyl group, pyrenyl group, perylenyl group, fluoranthenyl group and triphenylenyl group;
Aromatic heterocyclic groups, such as pyridyl group, pyrimidinyl group, triazinyl group, thienyl group, furyl group, pyrrolyl group, quinolyl group, isoquinolyl group, benzofuranyl group, benzothienyl group, indolyl group, carbazolyl group, benzoxazolyl group, benzothiazolyl group, quinoxalinyl group, benzimidazolyl group, pyrazolyl group, dibenzofuranyl group, dibenzothienyl group and carbolinyl group;
Aryloxy groups, such as phenyloxy group, biphenylyloxy group, naphthyloxy group, anthracenyloxy group and phenanthrenyloxy group;
Arylvinyl group, such as styryl group and naphthylvinyl group; and
Acyl groups, such as acetyl group and benzoyl group.

Here, the alkyl group having 1 to 6 carbon atoms, alkyloxy group having 1 to 6 carbon atoms and the alkenyl group may be in the form of either straight chains or branched chains. The above substituent may not have been substituted or may have been substituted with the substituent described above. Further, the above substituents may be present independently from each other without forming any ring, or may be bonded to each other via a single bond, a substituted or unsubstituted methylene group, an oxygen atom or a sulfur atom to form a ring.

### (C)

C represents an aromatic hydrocarbon group, an aromatic heterocyclic group or a condensed polycyclic aromatic group. When there are two groups C (when q = 2), the two groups C may be the same or different.

As the aromatic hydrocarbon group, aromatic heterocyclic group or condensed polycyclic aromatic group represented by C, there can be exemplified those that were exemplified above as the aromatic hydrocarbon groups, aromatic heterocyclic groups or condensed polycyclic aromatic groups represented by Ar¹ to Ar⁶ in the above-mentioned general formula (1).

These groups may be unsubstituted or may have a substituent. As the substituents, there can be exemplified those that were exemplified above as substituents that may be possessed by the aromatic hydrocarbon groups, aromatic heterocyclic groups or condensed polycyclic aromatic groups represented by Ar¹ to Ar⁶ in the above formula (1). The same also holds for the forms that can be assumed by the substituents.

### (D)

The groups D may be the same or different, and are hydrogen atoms, deuterium atoms, fluorine atoms, chlorine atoms, cyano groups, trifluoromethyl groups, alkyl groups having 1 to 6 carbon atoms, aromatic hydrocarbon groups, aromatic heterocyclic groups or condensed polycyclic aromatic groups. The alkyl group having 1 to 6 carbon atoms may be in the form of either a straight chain or branched chains.

As the alkyl group having 1 to 6 carbon atoms represented by D, there can be concretely exemplified methyl group, ethyl group, n-propyl group, isopropyl group, n-butyl group, isobutyl group, tert-butyl group, n-pentyl group, isopentyl group, neopentyl group and n-hexyl group.

As the aromatic hydrocarbon group, aromatic heterocyclic group or condensed polycyclic aromatic group represented by D, there can be exemplified those that were exemplified above as the aromatic hydrocarbon groups, aromatic heterocyclic groups or condensed polycyclic aromatic groups represented by Ar¹ to Ar⁶ in the above-mentioned general formula (1).

These groups may not be substituted or may have a substituent. As the substituent, there can be exemplified those that were exemplified above as substituents that may be possessed by the aromatic hydrocarbon groups, aromatic heterocyclic groups or condensed polycyclic aromatic groups represented by Ar¹ to Ar⁶ in the above formula (1). The same also holds for the forms that can be assumed by the substituents.

### (p, q)

The symbols p and q are such that, under the condition where the sum of p and q is 9, p is 7 or 8 and q is 1 or 2.

The groups D that are bonded in a plurality of number to the anthracene ring may be present independently from each other without forming any ring, or may be bonded to each other via a single bond, a substituted or unsubstituted methylene group, an oxygen atom or a sulfur atom to form a ring.

When q is 2, the groups C that are bonded in a plurality of number to the anthracene ring may be present independently from each other without forming any ring or may be bonded to each other via a single bond, a substituted or unsubstituted methylene group, an oxygen atom or a sulfur atom to form a ring.

### (Ar⁷ to Ar¹⁵)

Ar⁷ to Ar¹⁵ may be the same or different, and are aromatic hydrocarbon groups, aromatic heterocyclic groups or condensed polycyclic aromatic groups.

As the aromatic hydrocarbon groups, aromatic heterocyclic groups or condensed polycyclic aromatic groups represented by Ar⁷ to Ar¹⁵, there can be exemplified those that were exemplified above as the aromatic hydrocarbon groups, aromatic heterocyclic groups or condensed polycyclic aromatic groups represented by Ar¹ to Ar⁶ in the above-mentioned general formula (1).

These groups may not be substituted or may have a substituent. As the substituent, there can be exemplified those that were exemplified above as substituents that may be possessed by the aromatic hydrocarbon groups, aromatic heterocyclic groups or condensed polycyclic aromatic groups represented by Ar¹ to Ar⁶ in the above formula (1). The same also holds for the forms that can be assumed by the substituents.

### (R⁷ to R¹⁴)

R⁷ to R¹⁴ may be the same or different, and are hydrogen atoms, deuterium atoms, fluorine atoms, chlorine atoms, cyano groups, nitro groups, alkyl groups having 1 to 6 carbon atoms, cycloalkyl groups having 5 to 10 carbon atoms, alkenyl groups having 2 to 6 carbon atoms, alkyloxy groups having 1 to 6 carbon atoms, cycloalkyloxy groups having 5 to 10 carbon atoms, aromatic hydrocarbon groups, aromatic heterocyclic groups, condensed polycyclic aromatic groups or aryloxy groups. The alkyl groups having 1 to 6 carbon atoms, alkenyl groups having 2 to 6 carbon atoms and alkyloxy groups having 1 to 6 carbon atoms may be in the form of either straight chains or branched chains.

As the alkyl groups having 1 to 6 carbon atoms, cycloalkyl groups having 5 to 10 carbon atoms, alkenyl groups having 2 to 6 carbon atoms, alkyloxy groups having 1 to 6 carbon atoms or cycloalkyloxy groups having 5 to 10 carbon atoms represented by R⁷ to R¹⁴, there can be exemplified those that were exemplified above as the alkyl groups having 1 to 6 carbon atoms, cycloalkyl groups having 5 to 10 carbon atoms, alkenyl groups having 2 to 6 carbon atoms, alkyloxy groups having 1 to 6 carbon atoms or cycloalkyloxy groups having 5 to 10 carbon atoms represented by R¹ to R⁶ in the above general formula (1).

These groups may be unsubstituted or may have a substituent. As the substituent, there can be exemplified those that were exemplified above as substituents that may be possessed by alkyl group having 1 to 6 carbon atoms, cycloalkyl group having 5 to 10 carbon atoms or alkenyl group having 2 to 6 carbon atoms represented by R¹ to R⁶ in the above general formula (1). The same also holds for the forms that can be assumed by the substituents.

As the aromatic hydrocarbon groups, aromatic heterocyclic groups or condensed polycyclic aromatic groups represented by R⁷ to R¹⁴, there can be exemplified those that were exemplified above as the aromatic hydrocarbon groups, aromatic heterocyclic groups or condensed polycyclic aromatic groups represented by Ar¹ to Ar⁶ in the above general formula (1) .

These groups may be unsubstituted or may have a substituent. As the substituent, there can be exemplified those that were exemplified above as substituents that may be possessed by the aromatic hydrocarbon groups, aromatic heterocyclic groups or condensed polycyclic aromatic groups represented by Ar¹ to Ar⁶ in the above formula (1) . The same also holds for the forms that can be assumed by the substituents.

As the aryloxy groups represented by R⁷ to R¹⁴, there can be exemplified those that were exemplified above as aryloxy groups represented by R¹ to R⁶ in the above general formula (1) .

These groups may be unsubstituted or may have a substituent. As the substituent, there can be exemplified those that were exemplified above as substituents that may be possessed by the aromatic hydrocarbon groups, aromatic heterocyclic groups or condensed polycyclic aromatic groups represented by Ar¹ to Ar⁶ in the above general formula (1). The same also holds for the forms that can be assumed by the substituents.

R⁷ to R¹³ may be present independently from each other without forming any ring or may be bonded to each other via a single bond, a substituted or unsubstituted methylene group, an oxygen atom or a sulfur atom to form a ring.

### (X¹ to X⁴)

X¹ to X⁴ are, respectively, carbon atoms or nitrogen atoms, and any one only of X¹ to X⁴ is a nitrogen atom. The nitrogen atom, in this case, has neither a hydrogen atom nor a substituent R⁷ to R¹⁰. That is, R⁷ is not present when X¹ is a nitrogen atom, R⁸ is not present when X² is a nitrogen atom, R⁹ is not present when X³ is a nitrogen atom, or R¹⁰ is not present when X⁴ is a nitrogen atom.

### (Preferred embodiments of the anthracene derivative II)

Described below are preferred embodiments of the anthracene derivative II. In the description of the preferred embodiments, the groups that have not been specified to be substituted/unsubstituted may have a substituent or may be unsubstituted.

The anthracene derivative II is, preferably, represented by the above-mentioned general formula (2a), (2b) or (2c) and is, particularly preferably, represented by the above general formula (2b).

A³ is, preferably, a single bond, a divalent aromatic hydrocarbon group or a divalent condensed polycyclic aromatic group, more preferably, a divalent aromatic hydrocarbon group or a divalent condensed polycyclic aromatic group, and, specifically preferably, a divalent group obtained by removing two hydrogen atoms from benzene, biphenyl, naphthalene or phenanthrene. In the general formula (2c), A³ is, specifically preferably, a divalent aromatic hydrocarbon group.

The aromatic heterocyclic group represented by B is, preferably, a nitrogen-containing aromatic heterocyclic group, more preferably, a pyridyl group, a pyrimidinyl group, a pyrrolyl group, a quinolyl group, an isoquinolyl group, an indolyl group, a carbazolyl group, a benzoxazolyl group, a benzothiazolyl group, a quinoxalinyl group, a bezimidazolyl group, a pyrazolyl group or a carbolinyl group and, specifically preferably, a pyridyl group, a pyrimidinyl group, a quinolyl group, an isoqinolyl group, an indolyl group, a pyrazolyl group, a benzimidazolyl group or a carbolinyl group.

Of X¹ to X⁴, it is desired that X³ is a nitrogen atom.

D is, preferably, a hydrogen atom, a deuterium atom, a fluorine atom, a chlorine atom, a cyano group, a trifluoromethyl group or an alkyl group having 1 to 6 carbon atoms.

Ar⁷ is, preferably, an aromatic hydrocarbon group or a condensed polycyclic aromatic group and, more preferably, a phenyl group, a biphenyl group, a naphthyl group, a fluorenyl group or a phenanthrenyl group.

Ar⁸ and Ar⁹ maybe the same or different and are, preferably, aromatic hydrocarbon groups or condensed polycyclic aromatic groups and, more preferably, phenyl groups, naphthyl groups or phenanthrenyl groups.

Ar¹⁰ and Ar¹¹ may be the same or different and are, preferably, aromatic hydrocarbon groups or condensed polycyclic aromatic groups and, more preferably, phenyl groups, naphthyl groups or anthracenyl groups.

Ar¹² is, preferably, an aromatic hydrocarbon group or a condensed polycyclic aromatic group, more preferably, a phenyl group, a naphthyl group, an anthracenyl group or a phenanthrenyl group, and is, particularly preferably, a phenyl group.

Ar¹³ is, preferably, an aromatic hydrocarbon group, a nitrogen-containing aromatic heterocyclic group or a condensed polycyclic aromatic group and, more preferably, a phenyl group, a naphthyl group, a pyridyl group, a quinolyl group or a carbonyl group.

Ar¹⁴ and Ar¹⁵ may be the same or different and are, preferably, aromatic hydrocarbon groups, nitrogen-containing aromatic heterocyclic groups or condensed polycyclic aromatic groups and, more preferably, are phenyl groups, naphthyl groups, phenanthrenyl groups or carbolinyl groups. When these groups have a substituent, the substituent is, preferably, an aromatic hydrocarbon group, a condensed polycyclic aromatic group or an aromatic heterocyclic group and, more preferably, is a phenyl group, a naphthyl group, a phenanthrenyl group or a pyridyl group.

R⁷ to R¹⁰ may be the same or different and are, preferably, hydrogen atoms, aromatic hydrocarbon groups or aromatic heterocyclic groups and, more preferably, hydrogen atoms, phenyl groups or pyridyl groups.

R¹¹ to R¹³ may be the same or different and are, preferably, hydrogen atoms or deuterium atoms.

R¹⁴ is, preferably, a hydrogen atom or a deuterium atom.

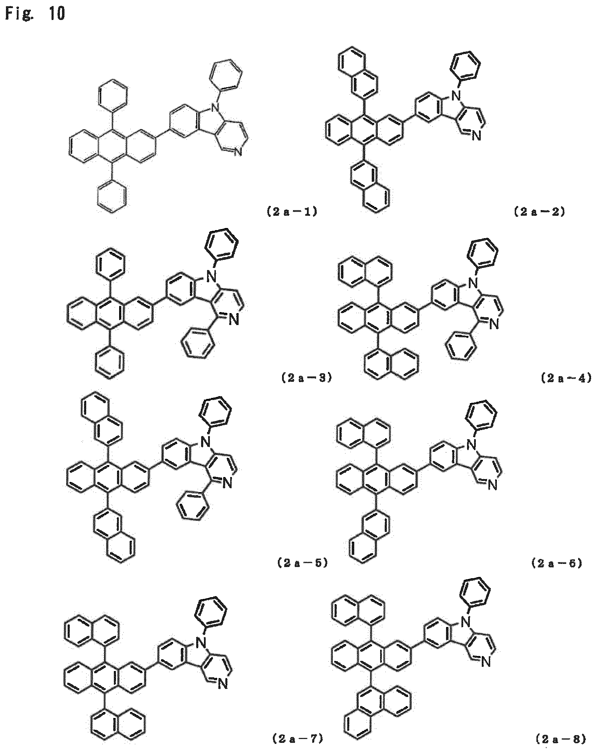

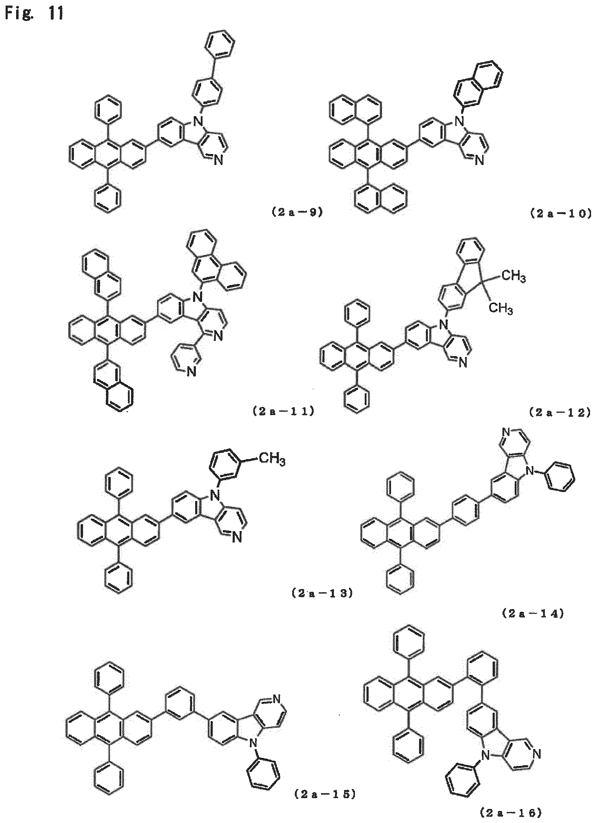

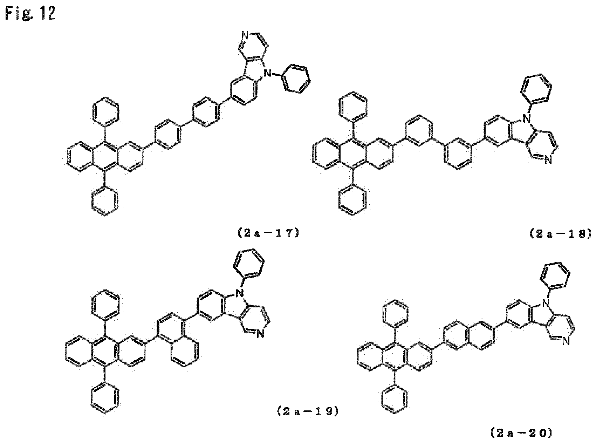

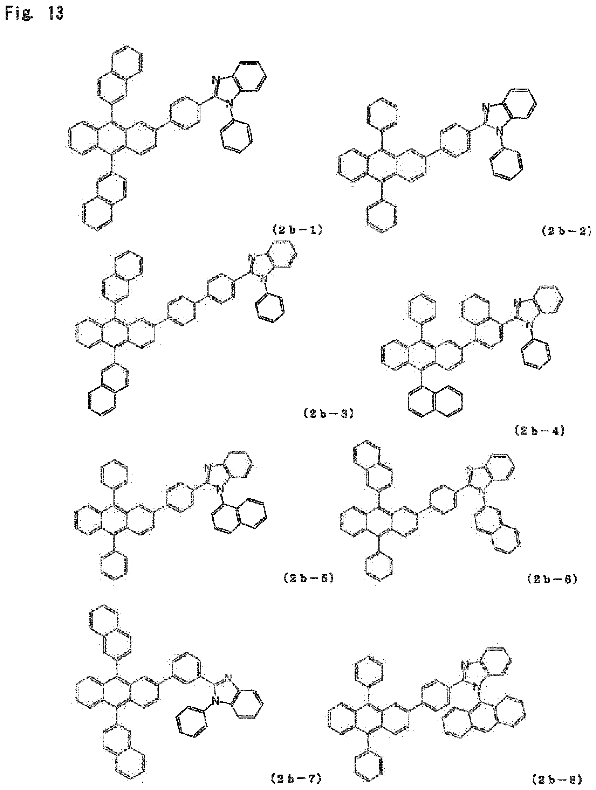

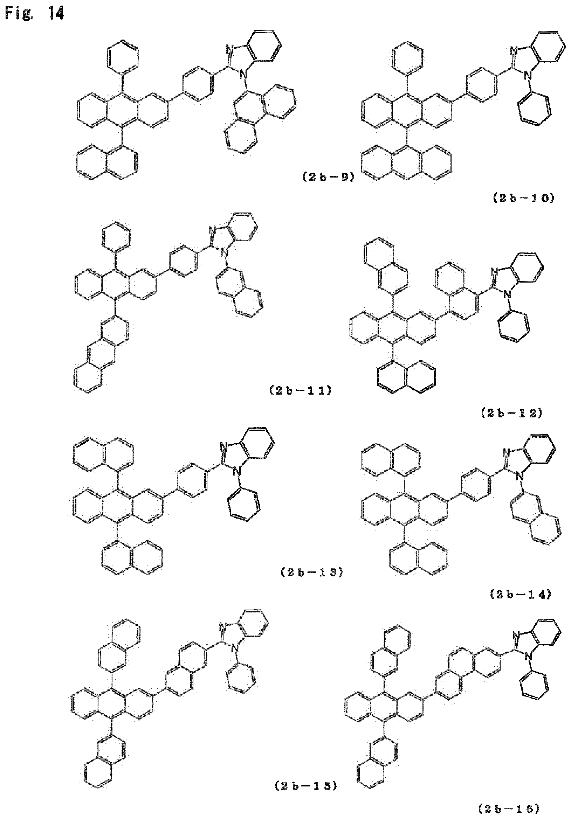

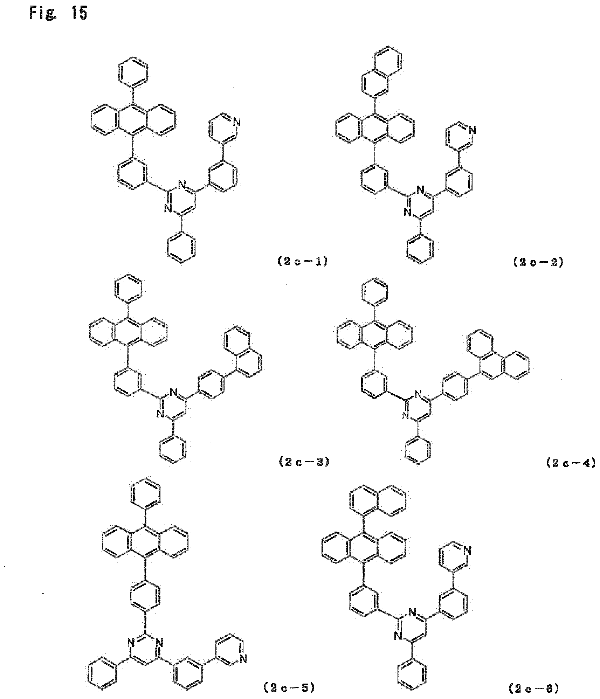

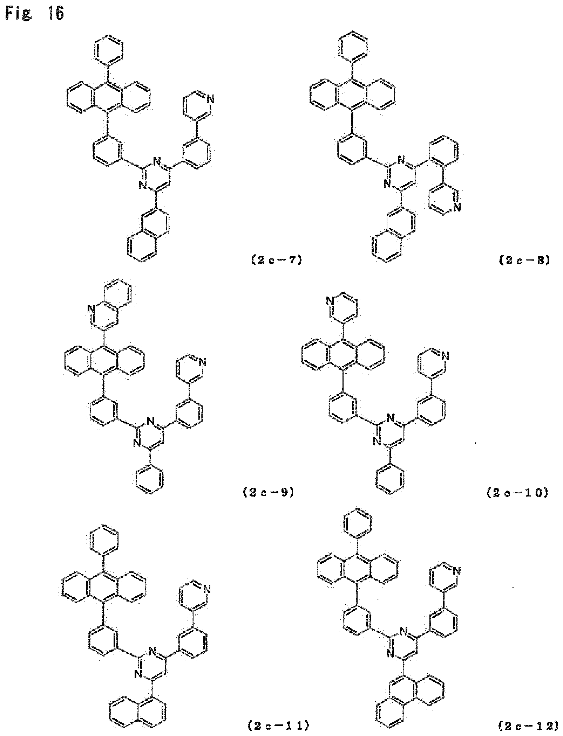

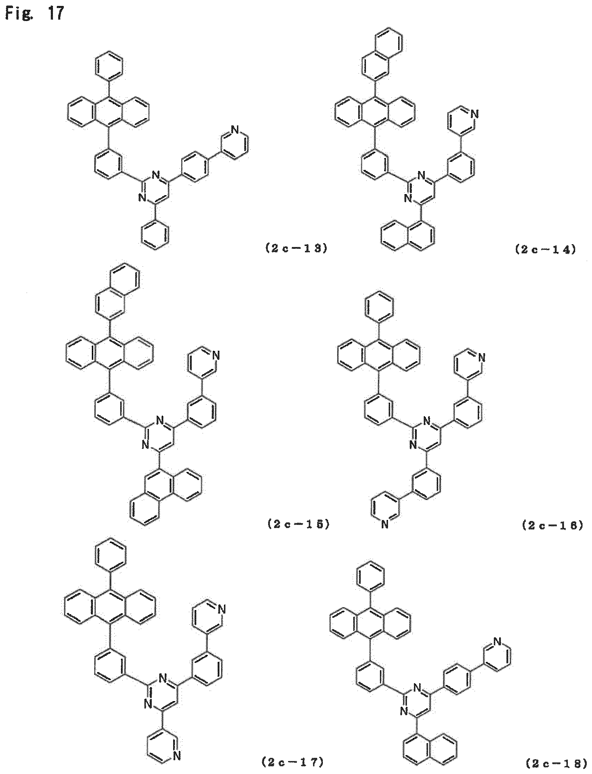

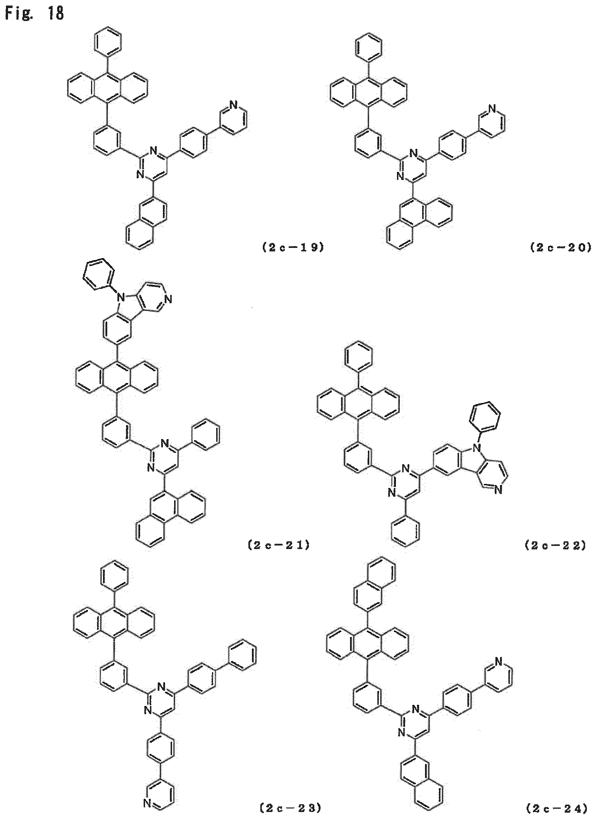

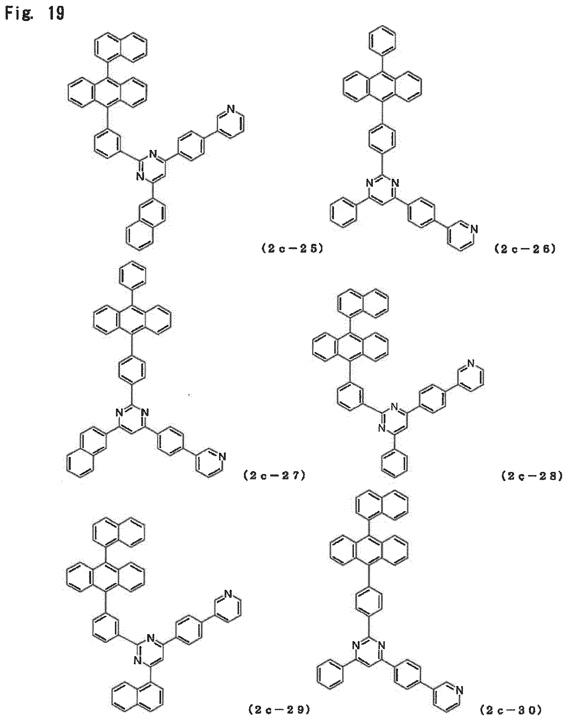

Figs. 10 to 19 concretely show preferred examples of the anthracene derivative II which, however, is in no way limited to these examples only. D represents a deuterium atom.

The above anthracene derivative II can be synthesized by the known methods (see patent documents 4 to 6).

The electron-transporting layer 7 may use the known electron-transporting materials being mixed together or simultaneously so far as they do not impair the effects of the invention. As the known electron-transporting materials, there can be used Alq₃ and Balq as well as metal complexes of quinolinol derivatives, various metal complexes, triazole derivatives, triazine derivatives, oxadiazole derivative, pyridine derivatives, pyrimidine derivatives, benzimidazole derivatives, thiadiazole derivatives, anthracene derivatives, carbodimide derivatives, quinoxaline derivatives, pyridoindole derivatives, phenanthroline derivatives and silole derivatives.

### <Electron injection layer 8>

An electron injection layer 8 may be provided on the electron-transporting layer 7. As the electron injection layer 8, there can be used an alkali metal salt such as lithium fluoride or cesium fluoride; an alkaline earth metal salt such as magnesium fluoride; a metal oxide such as aluminum oxide; or Liq. The electron injection layer 8, however, can be omitted if the electron-transporting layer and the cathode are preferably selected.

### <Cathode 9>

As the cathode 9, there is used an electrode material having a low work function, such as aluminum, or an alloy having a lower work function, such as magnesium-silver alloy, magnesium-indium alloy or aluminum-magnesium alloy.

### EXAMPLES

The invention will now be concretely described by way of Examples to which only, however, the invention is in no way limited.

### <Synthesis Example 1: Compound 33>

### Synthesis of an N,N'-bis(4'-diphenylamino-biphenyl-4-il)-N,N'-diphenyl-naphthalene-2,7-diamine;

Into a reaction vessel purged with a nitrogen atmosphere, there were added:

| | |
|---|---|
| 2,7-Dibromonaphthalene | 10.0 g, |
| (4'-Diphenylamino-biphenyl-4-il)-phenylamine | 31.0 g, |
| Tert-butoxysodium | 10.0 g and |
| Toluene | 330 ml, |
| followed by the addition of: | |
| Toluene solution of tert-butylphosphine | (10 wt%) |
| | 1.0 g and |
| Palladium acetate (II) | 0.2 g, |

and the mixture thereof was heated, refluxed and stirred for 3 hours. To the reaction solution were then added toluene and water, and an organic layer was picked up by the solution separation operation. The organic layer was dehydrated with an anhydrous magnesium sulfate and was then concentrated under reduced pressure to obtain a crude product. The crude product was refined by the column chromatography (carrier: silica gel, eluent: toluene/cyclohexane) and was, thereafter, refined again by the crystallization by using a mixed solvent of tetrahydrofurane/acetone. There was obtained 17.3 g of a yellowish white powder of the compound 33 (yield: 52.1%).

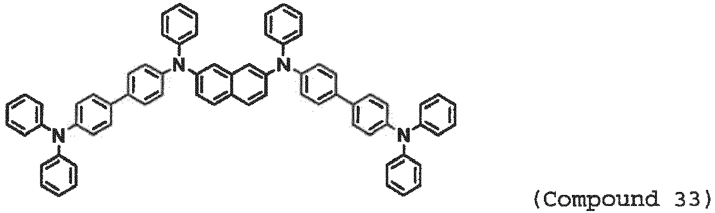

The obtained yellowish white powder was identified for its structure by the NMR. Fig. 1 shows the results of the ¹H-NMR (THF-d₈) measurement. The following 52 signals of hydrogen were detected by the ¹H-NMR (THF-d₈).
*δ* (ppm) = 7.85 (2H)
7.66 (8H)
7.42 (14H)
7.31 (10H)
7.25 (12H)
7.17 (6H)

The obtained compound was found for its glass transition point by using a high sensitivity differential scanning calorimeter (DSC3100S manufactured by Bruker AXS K.K.).

| | Glass transition point |
|---|---|
| Compound of Synthesis | |
| Example 1 (compound 33), | 149.8°C |

The arylamine compound I possessed a glass transition point of not lower than 100°C and, specifically, not lower than 130°C, and remained stable in the form of a thin film.

By using the thus obtained compound, a film was vapor-deposited in a thickness of 100 nm on an ITO substrate, and was measured for its work function by using an apparatus for measuring ionization potentials (Model PYS-202, manufactured by Sumitomo Heavy Industries, Ltd.).

| Compound of Synthesis | Work function |
|---|---|
| Example 1 (compound 33), | 5.61 eV |

As compared to the work function of 5.5 eV possessed by general hole-transporting materials such as NPD, TPD and the like, the arylamine compound I exhibits a favorable energy level and has a favorable hole-transporting capability.

### <Device Example 1>

An organic EL device of the structure shown in Fig. 2 was fabricated by, first, forming an ITO electrode as a transparent anode 2 on a glass substrate 1, and then by vapor-depositing thereon a hole injection layer 3, a hole-transporting layer 4, an electron-blocking layer 5, a luminous layer 6, an electron-transporting layer 7, an electron injection layer 8 and a cathode (aluminum electrode) 9 in this order.

Concretely, the glass substrate 1 having the ITO film of a thickness of 50 nm formed thereon was washed with an organic solvent. Thereafter, the ITO surface was washed by the treatment with UV/ozone. Thereafter, the glass substrate with the ITO electrode was placed in a vacuum evaporation machine, and the pressure therein was reduced down to 0.001 Pa or lower.

Next, a compound HIM-1 of the following structural formula was vapor-deposited so as to cover the transparent anode 2, to thereby form the hole injection layer 3 in a thickness of 5 nm.

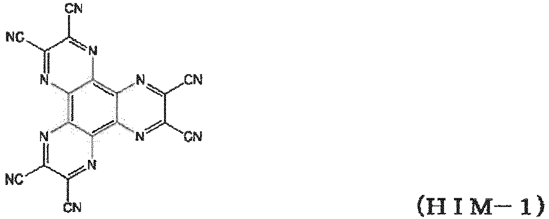

Next, the compound 33 of Synthesis Example 1 was vapor-deposited on the hole injection layer 3 to form the hole-transporting layer 4 in a thickness of 65 nm.

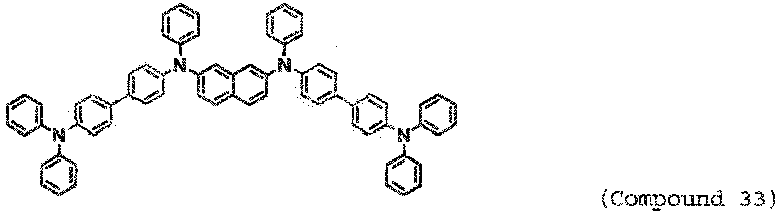

Next, a compound EBM-1 of the following structural formula was vapor-deposited on the hole-transporting layer 4 to thereby form the electron-blocking layer 5 in a thickness of 5 nm.

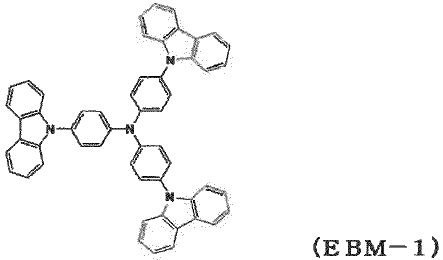

Next, a pyrene derivative EMD-1 of the following structural formula and an anthracene derivative 3-10 of the following structural formula were binary-vapor-deposited on the electron-blocking layer 5 at a deposition rate of EMD-1:derivative 3-10 = 5:95 to thereby form the luminous layer 6 in a thickness of 20 nm.

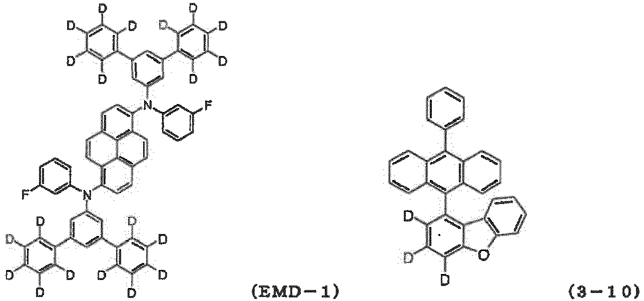

Next, an anthracene derivative 2b-1 of the following structural formula and a compound ETM-1 of the following structural formula were binary-vapor-deposited on the luminous layer 6 at a deposition rate of derivative 2b-1:ETM-1 = 50:50 to thereby form the electron-transporting layer 7 in a thickness of 30 nm.

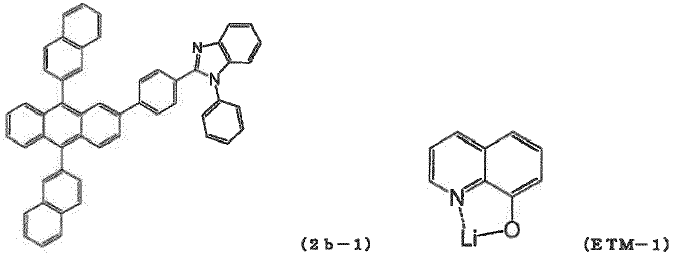

Next, the compound ETM-1 of the above structural formula was vapor-deposited on the electron-transporting layer 7 to thereby form the electron injection layer 8 in a thickness of 1 nm.

Finally, aluminum was deposited on the electron injection layer 8 to form the cathode 9 in a thickness of 100 nm.

The glass substrate on which the organic films and aluminum have been deposited were moved into a gloved box purged with dry nitrogen, and in which it was stuck to a sealing glass substrate by using an UV-curable resin to thereby obtain the organic EL device.

### <Comparative Device Example 1>

An organic EL device was fabricated under the same conditions as in Device Example 1 but using, as a material for forming the hole-transporting layer 4, a compound HTM-1 of the following structural formula instead of using the compound 33 of Synthesis Example 1.

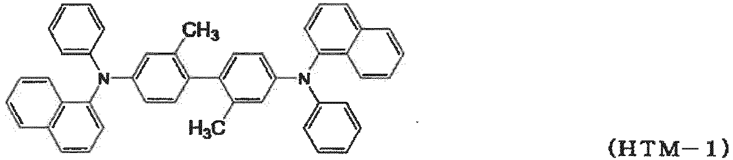

The EL devices fabricated in Device Example 1 and Comparative Device Example 1 were measured for their luminous characteristics when they were impressed with a DC voltage in the atmosphere at normal temperature. The results were as shown in Table 1.

The organic EL devices fabricated in Device Example 1 and Comparative Device Example 1 were measured for their service lives. Concretely, the organic EL devices were driven with a constant electric current, and were measured for their times from when they started emitting light at a luminance (initial luminance) of 2000 cd/m² until when their luminance decreased down to 1900 cd/m² (decreased down to 95% of the initial luminance of 100%: i.e., a reduction down to 95%). The results were as shown in Table 1.

**Table 1**

| | Hole-transporting layer | Voltage [V] (@10mA/cm²) | Luminance [cd/m²] (@10mA/cm²) | Luminous efficiency [cd/A] (@10mA/cm²) | Power efficiency [lm/W] (@10mA/cm²) | Service life [Hrs] (till decreased down to 95%) |
|---|---|---|---|---|---|---|
| Device Example 1 | Compound 33 | 3.68 | 560 | 5.60 | 4.77 | 375 |
| Comparative Device Example 1 | HTM-1 | 4.26 | 485 | 4.85 | 3.57 | 194 |

When an electric current was flown at a current density of 10 mA/cm², the driving voltage was 4.26 V in Comparative Device Example 1 but was as low as 3.68 V in Device Example 1.

The luminous efficiency was 4.85 cd/A in Comparative Device Example 1 but was as high as 5.60 cd/A in Device Example 1.

The power efficiency was 3.57 lm/W in Comparative Device Example 1 which, however, was greatly improved to be 4.77 lm/W in Device Example 1.

The service life was 194 hours in Comparative Example 1 but was greatly lengthened to be 375 hours in Device Example 1.

As will be obvious from the above results, as compared to the conventional organic EL device, the organic EL device of the present invention that uses the arylamine compound I represented by the general formula (1) realizes a low driving voltage, a high luminous efficiency and a long service life.

### Industrial Applicability:

As described above, the organic EL device of the present invention features a high luminous efficiency and power efficiency and enables the practical driving voltage to be lowered and the durability to be improved. Therefore, its use can be expanded to, for example, domestic appliances and illumination equipment.

### Description of Symbols:

- 1:: glass substrate
- 2:: transparent anode
- 3:: hole injection layer
- 4:: hole-transporting layer
- 5:: electron-blocking layer
- 6:: luminous layer
- 7:: electron-transporting layer
- 8:: electron injection layer
- 9:: cathode

## Claims

1. An organic EL device having at least an anode, a hole-transporting layer, a luminous layer, an electron-transporting layer and a cathode arranged in this order,
wherein said hole-transporting layer contains an arylamine compound represented by the following general formula (1), 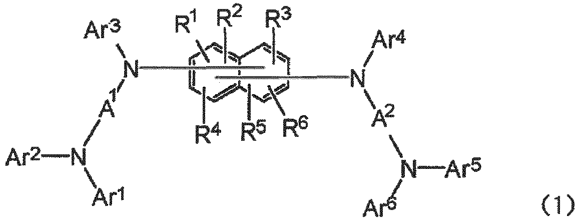 wherein,
Ar¹ to Ar⁶ may be the same or different, and are aromatic hydrocarbon groups, aromatic heterocyclic groups or condensed polycyclic aromatic groups,
A¹ and A² may be the same or different, and are divalent aromatic hydrocarbon groups, divalent aromatic heterocyclic groups or divalent condensed polycyclic aromatic groups, and
R¹ to R⁶ may be the same or different, and are hydrogen atoms, deuterium atoms, fluorine atoms, chlorine atoms, cyano groups, nitro groups, alkyl groups having 1 to 6 carbon atoms, cycloalkyl groups having 5 to 10 carbon atoms, alkenyl groups having 2 to 6 carbon atoms, alkyloxy groups having 1 to 6 carbon atoms, cycloalkyloxy groups having 5 to 10 carbon atoms, aromatic hydrocarbon groups, aromatic heterocyclic groups, condensed polycyclic aromatic groups or aryloxy groups.

2. The organic EL device according to claim 1, wherein said arylamine compound is represented by the following general formula (1a), 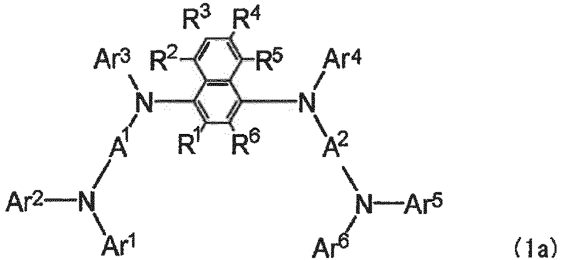 wherein,
Ar¹ to Ar⁶, A¹, A² and R¹ to R⁶ are as defined in the above general formula (1).

3. The organic EL device according to claim 1, wherein said arylamine compound is represented by the following general formula (1b), 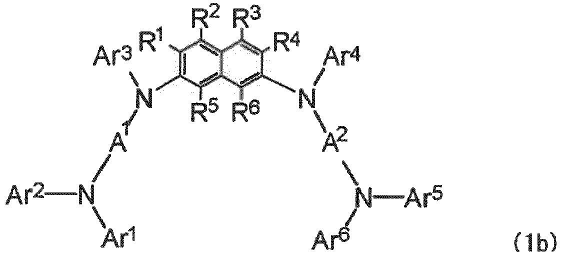 wherein,
Ar¹ to Ar⁶, A¹, A² and R¹ to R⁶ are as defined in the above general formula (1).

4. The organic EL device according to claim 1, wherein said arylamine compound is represented by the following general formula (1c), 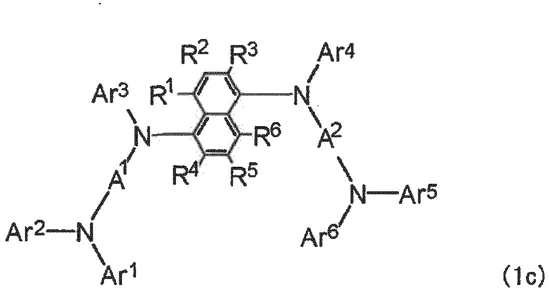 wherein,
Ar¹ to Ar⁶, A¹, A² and R¹ to R⁶ are as defined in the above general formula (1).

5. The organic EL device according to claim 1, wherein said electron-transporting layer contains an anthracene derivative represented by the following general formula (2), 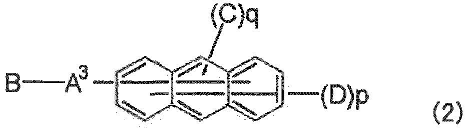 wherein,
A³ is a divalent aromatic hydrocarbon group, a divalent aromatic heterocyclic group, a divalent condensed polycyclic aromatic group or a single bond,
B is an aromatic heterocyclic group,
C is an aromatic hydrocarbon group, an aromatic heterocyclic group or a condensed polycyclic aromatic group, and when there are two Cs, the two Cs may be the same or different,
D may be the same or different, and is a hydrogen atom, a deuterium atom, a fluorine atom, a chlorine atom, a cyano group, a trifluoromethyl group, an alkyl group having 1 to 6 carbon atoms, an aromatic hydrocarbon group, an aromatic heterocyclic group or a condensed polycyclic aromatic group, and
p is 7 or 8, and q is 1 or 2 under the condition that the sum of p and q is 9.

6. The organic EL device according to claim 5, wherein said anthracene derivative is represented by the following general formula (2a), 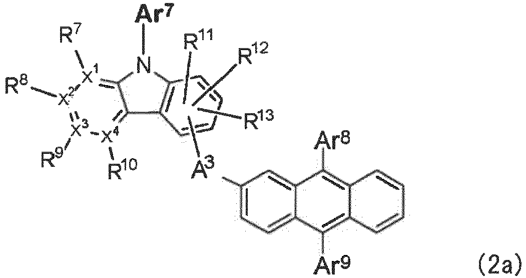 wherein,
A³ is a divalent aromatic hydrocarbon group, a divalent aromatic heterocyclic group, a divalent condensed polycyclic aromatic group or a single bond,
Ar⁷ to Ar⁹ may be the same or different, and are aromatic hydrocarbon groups, aromatic heterocyclic groups or condensed polycyclic aromatic groups,
R⁷ to R¹³ may be the same or different, and are hydrogen atoms, deuterium atoms, fluorine atoms, chlorine atoms, cyano groups, nitro groups, alkyl groups having 1 to 6 carbon atoms, cycloalkyl groups having 5 to 10 carbon atoms, alkenyl groups having 2 to 6 carbon atoms, alkyloxy groups having 1 to 6 carbon atoms, cycloalkyloxy groups having 5 to 10 carbon atoms, aromatic hydrocarbon groups, aromatic heterocyclic groups, condensed polycyclic aromatic groups or aryloxy groups, and may be bonded to each other via a single bond, a methylene group, an oxygen atom or a sulfur atom to form a ring, and
X¹ to X⁴ are, respectively, carbon atoms or nitrogen atoms, only any one of X¹ to X⁴ being the nitrogen atom and, in this case, the nitrogen atom does not have any R⁷ to R¹⁰ of hydrogen atom or a substituent.

7. The organic EL device according to claim 5, wherein said anthracene derivative is represented by the following general formula (2b), 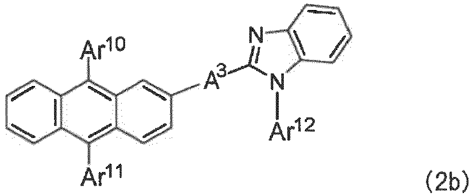 wherein,
A³ is a divalent aromatic hydrocarbon group, a divalent aromatic heterocyclic group, a divalent condensed polycyclic aromatic group or a single bond, and
Ar¹⁰ to Ar¹² may be the same or different, and are aromatic hydrocarbon groups, aromatic heterocyclic groups or condensed polycyclic aromatic groups.

8. The organic EL device according to claim 5, wherein said anthracene derivative is represented by the following general formula (2c), 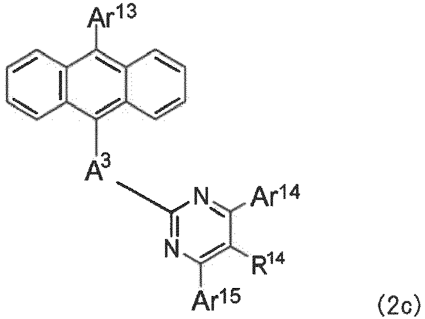 wherein,
A³ is a divalent aromatic hydrocarbon group, a divalent aromatic heterocyclic group, a divalent condensed polycyclic aromatic group or a single bond,
Ar¹³ to Ar¹⁵ may be the same or different, and are aromatic hydrocarbon groups, aromatic heterocyclic groups or condensed polycyclic aromatic groups, and
R¹⁴ is a hydrogen atom, a deuterium atom, a fluorine atom, a chlorine atom, a cyano group, a nitro group, an alkyl group having 1 to 6 carbon atoms, a cycloalkyl group having 5 to 10 carbon atoms, an alkenyl group having 2 to 6 carbon atoms, an alkyloxy group having 1 to 6 carbon atoms, a cycloalkyloxy group having 5 to 10 carbon atoms, an aromatic hydrocarbon group, an aromatic heterocyclic group, a condensed polycyclic aromatic group or an aryloxy group.

9. The organic EL device according to claim 1, wherein said luminous layer contains a blue light-emitting dopant.

10. The organic EL device according to claim 9, wherein said blue light-emitting dopant is a pyrene derivative.

11. The organic EL device according to claim 1, wherein said luminous layer contains an anthracene derivative.

12. The organic EL device according to claim 11, wherein said luminous layer contains the anthracene derivative as a host material.

13. The organic EL device according to claim 12, wherein said anthracene derivative is represented by the following general formula (3), 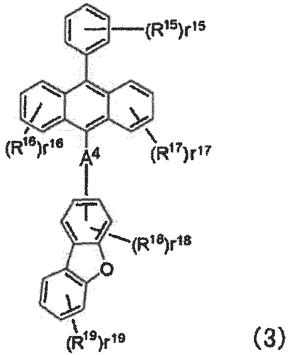 wherein,
R¹⁵ to R¹⁹ may be the same or different, and are deuterium atoms, alkyl groups having 1 to 30 carbon atoms, alkenyl groups having 2 to 30 carbon atoms, alkinyl groups having 2 to 30 carbon atoms, cycloalkyl groups having 3 to 30 carbon atoms, cycloalkenyl groups having 5 to 30 carbon atoms, alkyloxy groups having 1 to 30 carbon atoms, aryloxy groups having 6 to 30 carbon atoms, alkylthio groups having 1 to 30 carbon atoms, arylthio groups having 5 to 30 carbon atoms, alkylamino groups having 1 to 30 carbon atoms, arylamino groups having 5 to 30 carbon atoms, aryl groups having 6 to 50 carbon atoms, aromatic heterocyclic groups having 2 to 50 carbon atoms, cyano groups, nitro groups, halogen atoms, amino groups, hydroxy groups or -CO-R²⁰ groups,
R²⁰ is a hydrogen atom, a hydroxy group, an alkyloxy group having 1 to 6 carbon atoms or an aryloxy group having 6 to 30 carbon atoms,
A⁴ is a divalent aromatic hydrocarbon group, a divalent condensed polycyclic aromatic group or a single bond,
r¹⁵ is an integer of 0 to 5, r¹⁶ r¹⁷ and r¹⁹ are, respectively, integers of 0 to 4, and r¹⁸ is an integer of 0 to 3, and
when R¹⁵ to R¹⁹ are bonded to the same benzene ring plurally, the plurality of the groups that are bonded may be the same or different.

14. An organic EL device having an anode, a hole-transporting layer, an electron-blocking layer, a luminous layer, an electron-transporting layer and a cathode arranged in this order, wherein
said electron-blocking layer contains an arylamine compound represented by the following general formula (1), 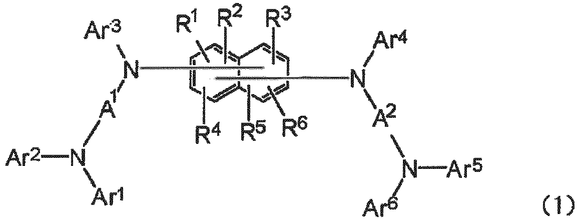 wherein,
Ar¹ to Ar⁶ may be the same or different, and are aromatic hydrocarbon groups, aromatic heterocyclic groups or condensed polycyclic aromatic groups,
A¹ and A² may be the same or different, and are divalent aromatic hydrocarbon groups, divalent aromatic heterocyclic groups or divalent condensed polycyclic aromatic groups, and
R¹ to R⁶ may be the same or different, and are hydrogen atoms, deuterium atoms, fluorine atoms, chlorine atoms, cyano groups, nitro groups, alkyl groups having 1 to 6 carbon atoms, cycloalkyl groups having 5 to 10 carbon atoms, alkenyl groups having 2 to 6 carbon atoms, alkyloxy groups having 1 to 6 carbon atoms, cycloalkyloxy groups having 5 to 10 carbon atoms, aromatic hydrocarbon groups, aromatic heterocyclic groups, condensed polycyclic aromatic groups or aryloxy groups.

15. An organic EL device having an anode, a hole injection layer, a hole-transporting layer, a luminous layer, an electron-transporting layer and a cathode arranged in this order, wherein
said hole injection layer contains an arylamine compound represented by the following general formula (1), 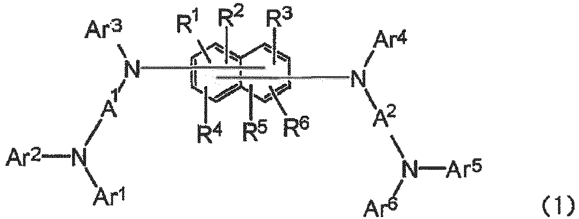 wherein,
Ar¹ to Ar⁶ may be the same or different, and are aromatic hydrocarbon groups, aromatic heterocyclic groups or condensed polycyclic aromatic groups,
A¹ and A² may be the same or different, and are divalent aromatic hydrocarbon groups, divalent aromatic heterocyclic groups or divalent condensed polycyclic aromatic groups, and
R¹ to R⁶ may be the same or different, and are hydrogen atoms, deuterium atoms, fluorine atoms, chlorine atoms, cyano groups, nitro groups, alkyl groups having 1 to 6 carbon atoms, cycloalkyl groups having 5 to 10 carbon atoms, alkenyl groups having 2 to 6 carbon atoms, alkyloxy groups having 1 to 6 carbon atoms, cycloalkyloxy groups having 5 to 10 carbon atoms, aromatic hydrocarbon groups, aromatic heterocyclic groups, condensed polycyclic aromatic groups or aryloxy groups.

16. An organic EL device having an anode, a hole-transporting layer, a luminous layer, an electron-transporting layer and a cathode arranged in this order, wherein
said luminous layer contains an arylamine compound represented by the above-mentioned general formula (1), 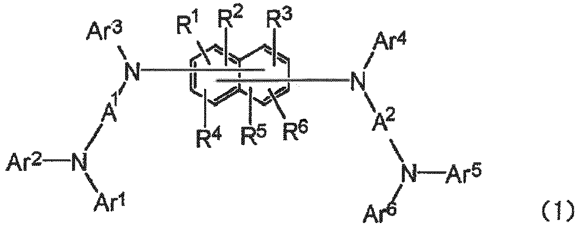 wherein,
Ar¹ to Ar⁶ may be the same or different, and are aromatic hydrocarbon groups, aromatic heterocyclic groups or condensed polycyclic aromatic groups,
A¹ and A² may be the same or different, and are divalent aromatic hydrocarbon groups, divalent aromatic heterocyclic groups or divalent condensed polycyclic aromatic groups, and
R¹ to R⁶ may be the same or different, and are hydrogen atoms, deuterium atoms, fluorine atoms, chlorine atoms, cyano groups, nitro groups, alkyl groups having 1 to 6 carbon atoms, cycloalkyl groups having 5 to 10 carbon atoms, alkenyl groups having 2 to 6 carbon atoms, alkyloxy groups having 1 to 6 carbon atoms, cycloalkyloxy groups having 5 to 10 carbon atoms, aromatic hydrocarbon groups, aromatic heterocyclic groups, condensed polycyclic aromatic groups or aryloxy groups.
